# EUROPEAN PATENT SPECIFICATION

(11) **EP 4 442 207 B1**
(45) Date of publication and mention of the grant of the patent: **08.10.2025**
(21) Application number: 23167214.8
(22) Date of filing: 07.04.2023
(51) Int. Cl.: A61B 8/08, A61B 8/00, G16H 50/20

(54) **DEVICE FOR GUIDING A USER IN ULTRASOUND ASSESSMENT OF AN ORGAN**
VORRICHTUNG ZUR FÜHRUNG EINES BENUTZERS BEI DER ULTRASCHALLBEURTEILUNG EINES ORGANS
DISPOSITIF DE GUIDAGE D'UN UTILISATEUR DANS L'ÉVALUATION PAR ULTRASONS D'UN ORGANE

(43) Date of publication of application: 09.10.2024
(73) Proprietor: Diagnoly, 69003 Lyon (FR)
(72) Inventor: VOZNYUK, Ivan, 69100 Villeurbanne (FR); DUPONT, Camille, 69009 Lyon (FR)
(74) Representative: Icosa

(56) References cited:
- EP-A1- 3 964 136
- US-A1- 2020 388 391
- US-A1- 2021 259 660
- US-A1- 2022 338 842
- LU ALLEN ET AL: "Detecting anomalies from echocardiography using multi-view regression of clinical measurements", 2018 IEEE 15TH INTERNATIONAL SYMPOSIUM ON BIOMEDICAL IMAGING (ISBI 2018), IEEE, 4 April 2018 (2018-04-04), pages 1504 - 1508, XP033348435, [retrieved on 20180523], DOI: 10.1109/ISBI.2018.8363858

## Description

### FIELD OF INVENTION

The present invention relates to the field of medical imaging. In particular, the invention relates to a device and method for guiding a user in ultrasound assessment of an organ so as to perform a screening evaluation of said organ during a medical examination.

### BACKGROUND OF INVENTION

Ultrasound imaging is a non-invasive diagnostic technique used to image the inside of the body, using ultrasound waves. There are many uses of ultrasound imaging, including imaging of the heart, blood vessels, eyes, thyroid, brain, breast, abdominal organs, skin, and muscles.
In order to assess a specific organ, medical guidelines have defined lists of first criteria that may be verified on ultrasound images. These first criteria are indicative of an organ having a defect. Physicians may derive their diagnosis from the verification of the first criteria in at least one ultrasound image.

Such first criteria may be verified by analyzing remarkable elements present in the ultrasound images. A remarkable element is an ultrasound structural feature of the ultrasound image that contains information that may directly or indirectly be used to verify a first criterion.

One of the most common uses of ultrasound is during pregnancy, to monitor the growth and development of the fetus by detecting fetal abnormalities. A fetal abnormality is a defect in the fetus of a genetic and/or structural nature that can cause significant fetal health and developmental complications, maternal complicated delivery and lead to complex postnatal medical interventions and adverse outcomes.

Detection of fetal anomalies allows to anticipate a tailored inborn birth, prompt medical care or surgical treatment in specialized centers at birth or in the neonatal period, as well as to provide an adequate information to the parents. For all of these reasons, the ability to detect fetal complications prenatally is critical.

However, despite the progress in equipment, up to 52.8% of prenatal cardiac pathologies are unfortunately not detected today. This high rate is due to the fact that the examination is highly "operator-dependent" as well as a "patient-dependent". As a result, these factors can generate errors in prenatal diagnosis and an overload of work involving a lack of time for health care staff.

According to specialists, fetal heart disease is not always screened antenatally for 3 main reasons: (1) in 49% of cases, lack of skill in adapting ultrasound images (the ultrasound images obtained by the operator are not sufficient to make the correct diagnosis); (2) in 31% of the cases, lack of experience in diagnostics (the images obtained are good and the prenatal pathology is visible but not recognized by the operator); and finally (3), in 20% of cases, pathologies cannot be detected because they are not visible on the ultrasound images.

Different computer-implemented methods have been proposed to help the users to assess ultrasound images of organs and analyze these images in order to detect a large spectrum of abnormalities. More precisely, these methods may be configured to process each image independently to predict a probability of an organ having at least one abnormality related to a predetermined first criterion.

For instance, a binary system may be configured to detect as a remarkable element in a given ultrasound image a blood vessel in a specific position with respect to the heart. The first criterion to be verified is the absence of said blood vessel at this specific position. Indeed, if this blood vessel is detected at this specific position, it means that there is a malformation that might cause symptoms such as high blood pressure in the right side of the heart. If the ultrasound image contains an artifact that makes the area where the blood vessel is supposed to be detected impossible to analyze, the system may output a high probability of absence of the blood vessel because it interprets the artifact as the absence of said blood vessel when in facts it is hidden by the artifact. In the end, the binary system will output an unreliable result because its decision is based on a corrupted image.

As another example, a binary system may be configured to detect as a remarkable element in a given ultrasound image a discontinuity in the heart interventricular septum. The first criterion to be verified is a septal defect. Indeed, a hole in the septum causes an increased amount of blood that flows toward the lungs. Over time, it may cause damage to the blood vessels in the lungs and ultimately lead to high blood pressure and heart failure. However, a vertical angle between the probe and the septum may cause a dropout of echoes throughout the septum, hence forming a shadow resembling the appearance of hole in the septum, even in normal hearts. The binary system interpretation of the septum defect in these viewpoint conditions may lead to overdiagnosis of interventricular septal defects.

In all of the examples above, the system relies on the analysis of individual ultrasound images. Therefore, some prediction may be biased due to an unsuitable viewpoint of the ultrasound image. Moreover, ultrasound images represent the organ state at a specific time point. Therefore, these systems are unable to assess temporal criteria, such as the regular opening and closing of a valve. Additionally, such systems are not configured to reflect on the accuracy of their prediction. Therefore, the physician has no way to verify the accuracy of the system deduction unless he analyzes himself the ultrasound image. This lack of reliability may cause a huge time loss or even worse a wrong diagnosis from the physician.

A neural network based system configured to analyse a set of multiple ultrasound images and to generate two global scores, said global scores in combination are indicative of an impossibility to assess a global criterion and a global presence / absence of a defect based on the set of the ultrasound images, is known from document US 2021/259660.

Therefore, the problem solved by the present invention is to provide a device and a method with an improved prediction accuracy and a greater robustness with respect to errors prediction and assessment of temporal criteria.

### SUMMARY

The present invention related to a device for guiding a user in ultrasound assessment of an organ to perform an evaluation of said organ during a medical examination, said ultrasound assessment being based on at least two ultrasound images resulting from at least a partial reflection of ultrasound waves by said organ, said device comprising at least one processor configured to:
- for each ultrasound image:
   - receive as an input, for each ultrasound image of the at least two ultrasound images, a list of remarkable elements associated to the ultrasound image, wherein each remarkable element is associated with at least one remarkable information among:
      o an observability of the remarkable element or an unobservability of the remarkable element,
      o an abnormal aspect of the remarkable element or a non-abnormal aspect of the remarkable element,
      o a normal aspect of the remarkable element or a non-normal aspect of the remarkable element, and
      o a measurement performed on the remarkable element,
   - generate and output a list of first criteria indicative of a health status of the organ, wherein each first criterion of the list of first criteria is associated with at least two first scores, each first score being generated using said at least one remarkable information associated to the at least one remarkable element, said at least two first scores being such that a combination of the at least two first scores provides information on an impossibility to assess the first criterion, a presence of a defect and an absence of a defect,
- generate and output a list of global criteria indicative of a health status of the organ assessed over said at least two ultrasound images, wherein each global criterion of the list of global criteria is associated with at least two global scores, said at least two global scores being such that a combination of the at least two global scores provides an information on a global impossibility to assess the global criterion, a global presence of a defect and a global absence of a defect, wherein the list of global criteria is generated using a feed-forward network of arithmetic operators of linear or non-linear nature which maps at least two of said generated lists of first criteria, and/or at least two lists of remarkable elements relative to the at least two ultrasound images from which said at least two lists of first criteria have been generated, to the at least two global scores.

According to the invention, a health status of the organ corresponds to a presence or an absence of a defect in the organ.

A first advantage of the device of the invention concerns the nature of the first scores and global scores. Indeed, the first scores and global scores respectively convey an impossibility to assess the first criterion and global criterion. This can help improving the accuracy of the assessments by reducing errors made by the system. If an image is impossible to assess, it can be flagged for review, and the presence or absence of a defect result can be double-checked to ensure its accuracy. If the whole set of ultrasound images is impossible to assess, the system can notify the physician that he needs to acquire more ultrasound images.

As a result, the device only provides predictions when it has enough information to do so with a high level of confidence. When it encounters data that is too ambiguous or incomplete, it will simply say "I do not know" rather than making a guess. This reduces the likelihood of false positives or false negatives and ensures that healthcare professionals can rely on the algorithm's predictions with a high degree of confidence.

Therefore, the combination of three information in the first scores and global scores can improve the efficiency of the diagnostic process. By automating the detection process and the calculation of the scores, physicians can save time and resources when evaluating images. They can be more confident in the results outputted by the device without having to double-check the ultrasound images analysis.

Another advantage of the invention relates to the global scores. Indeed, they have a reduced error rate compared to first scores calculated on individual ultrasound images. The global scores provide a summary of the three types of global information across the whole set of ultrasound images. As a result, the system is more robust with respect to prediction errors. Even when faced with imperfect data, such as ultrasound images of poor quality, it can still produce reliable results.

According to one embodiment, at least one global criterion is a temporal criterion indicative of a defect in a periodic evolution of the organ and the at least two global scores are determined based on the evolution in time of the at least one remarkable element across the at least two ultrasound images.

This temporal criterion is particularly advantageous for the assessment of organs that, for their nature, modify periodically their configuration such as the heart, the lungs, and the like. More precisely, the temporal criterion is particularly advantageous to verify if a valve is regularly opening and closing, which is impossible to verify in isolated ultrasound images.

According to one embodiment, each first criterion of the list of first criteria is further associated with a first severity score representative of a magnitude of the defect.

By quantifying the severity of the abnormal aspect, the severity score can provide additional information to the physician that may not be immediately apparent from a visual inspection of the ultrasound image. A high severity score will catch more the attention of the physician and make him more attentive during his analysis of the corresponding ultrasound image.

According to one embodiment, each global criterion of the list of global criteria is further associated with a global severity score representative of a magnitude of the defect assessed on the at least two ultrasound images.

A global severity score can help to more accurately identify and diagnose abnormalities or pathologies within the organ. Advantageously, by quantifying the severity of the abnormality, the global severity score can provide additional information that may not have been detected by the physician during his own analysis of the ultrasound images.

According to one embodiment, the at least one remarkable information is comprised in at least one remarkable score.

According to one embodiment, each first criterion of the list of first criteria is associated with three mutually exclusive first scores, including:
- an assessability score, representative of the impossibility to assess the first criterion,
- a presence score, representative of the presence of a defect, and
- an absence score, representative of the absence of a defect.

The first scores outputted by the transformation module may be available for the physician to consult. These first scores provide a summary of the three types of first information and can help the physician verifying and/or deciding on the assessment of a criterion on a given ultrasound image. Indeed, advantageously the first scores can make the ultrasound data more accessible and easier to interpret even for non-expert users, thus improving its usefulness in decision-making.

Defining one first information per first score is the easiest way to convey the first information to the physician. He only needs to consider the three first scores independently to draw his conclusions on a specific ultrasound image. The presence and absence scores, may be interpreted by the physician so as to evaluate the probability of presence or absence of a defect in the organ on a given ultrasound image based on the remarkable elements analyzed for this criterion. The assessability score may be interpreted by the physician so as to evaluate the sufficiency or insufficiency of the remarkable information associated with the at least one remarkable element used to assess the criterion. In other words, the assessability score may be used to double check the correctness of the presence and absence scores. Indeed, a first score conveying a high probability of presence or absence of a defect combined with an assessability score conveying an impossibility to assess the criterion is unreliable and should not be taken into account by the physician to draw his diagnosis.

According to one embodiment, each first criterion of the list of first criteria is associated with two first scores:
- a defect score representative of the presence of a defect and the absence of a defect, said defect score being comprised between a lower score bound and an upper score bound, the lower score bound being representative of a high probability of an absence of a defect and the upper score bound being representative of a high probability of a presence of a defect, and
- an assessability score, being representative of the impossibility to assess the first criterion.

According to one embodiment, each global criterion of the list of global criteria is associated with two global scores:
- a global defect score representative of the global absence of a defect and the global presence of a defect, said global defect score being comprised between a global lower score bound and a global upper score bound, the global lower score bound being representative of a high probability of an absence of a defect across the at least two ultrasound images and the upper score bound being representative of a high probability of a presence of a defect across the at least two ultrasound images, and
- a global assessability score, representative of the global impossibility to assess the global criterion.

According to one embodiment, the generation of the list of global criteria comprises implementing a 1-dimensional convolutional neural network configured to simultaneously process the outputted lists of first criteria and/or the lists of remarkable elements relative to the at least two ultrasound images.

According to one embodiment, the generation of the list of global criteria comprises implementing a recurrent convolutional neural network configured to iteratively process the outputted lists of first criteria and/or the lists of remarkable elements relative to the at least two ultrasound images.

There are several advantages to giving first scores as an input to a convolutional neural network (CNN) instead of directly giving raw data. Indeed, first scores represent a compact summary of complex raw data, thus reducing the dimensionality of the input. This helps to reduce the number of parameters in the CNN and improve its computational efficiency. Moreover, the first scores are less sensitive to noise or variations that may be present in the raw data, as they represent a more robust and generalized representation of the underlying first information. Furthermore, the first scores can be more easily transferred across different datasets or applications, as they often represent a more generalizable representation of the underlying first information that is less dependent on the specific characteristics of the input data.

As a result, the system is extremely light in terms of memory, making it easy to use on a wide range of devices.

According to another aspect, the invention relates to a computer-implemented method for guiding a user in ultrasound assessment of an organ to perform a diagnosis or screening evaluation of said organ during a medical examination, said ultrasound assessment being based on at least two ultrasound image resulting from at least a partial reflection of ultrasound waves by said organ, said method comprising:
- for each ultrasound image:
   - receiving a list of remarkable elements associated to the at least two ultrasound images, wherein each remarkable element is associated with at least one remarkable information among:
      o an observability of the remarkable element or an unobservability of the remarkable element,
      o an abnormal aspect of the remarkable element or a non-abnormal aspect of the remarkable element,
      o a normal aspect of the remarkable element or a non-normal aspect of the remarkable element, and
      o a measurement performed on the remarkable element,
   - generating and outputting a list of first criteria indicative of a health status of the organ, wherein each first criterion of the list of first criteria is associated with at least two first scores, each first score being generated using said at least one remarkable information associated to the at least one remarkable element, said at least two first scores being such that a combination of the at least two first scores provides information on an impossibility to assess the first criterion, a presence of a defect, and an absence of a defect,
- generating and outputting a list of global criteria indicative of a health status of the organ assessed over said at least two ultrasound images, wherein each global criterion of the list of global criteria, said at least two global scores being such that a combination of the at least two global scores provides information on a global impossibility to assess the global criterion, a global presence of a defect and a global absence of a defect, wherein the list of global criteria is generated using a feed-forward network of arithmetic operators of linear or non-linear nature which maps at least two of said generated lists of first criteria, and/or at least two lists of remarkable elements relative to the at least two ultrasound images from which said at least two lists of first criteria have been generated, to the at least two global scores.

According to another aspect, the invention relates to a computer program product for guiding a user in ultrasound assessment of an organ, the computer program product comprising instructions which, when the program is executed by a computer, cause the computer to automatically carry out the method described previously.

According to another aspect, the invention relates to a computer-readable storage medium comprising instructions which, when executed by a computer, cause the computer to carry out the method for guiding a user in ultrasound assessment of an organ as described previously.

### BRIEF DESCRIPTION OF THE DRAWINGS

**Figure 1** is a block diagram representation of the device according to an embodiment of the invention,
**Figure 2** is an ultrasound image comprising abnormal remarkable elements analyzed for the fourth vessel criterion and the remarkable scores of the remarkable elements,
**Figure 3** is an ultrasound image comprising normal remarkable elements analyzed for the fourth vessel criterion and the remarkable scores of the remarkable elements,
**Figure 4** is an ultrasound image comprising inobservable remarkable elements analyzed for the fourth vessel criterion and the remarkable scores of the remarkable elements,
**Figure 5** is an ultrasound image comprising abnormal and non-normal remarkable elements analyzed for the septum defect criterion and the remarkable scores of the remarkable elements,
**Figure 6** is an ultrasound image comprising normal and non-abnormal remarkable elements analyzed for the septum defect first criterion and the remarkable scores of the remarkable elements,
**Figure 7** is an ultrasound image comprising unobservable remarkable elements analyzed for the septum defect first criterion and the remarkable scores of the remarkable elements,
**Figure 8** is an ultrasound image comprising two measurement remarkable elements analyzed for ventricles asymmetry first criterion and the values of the measurement performed on the remarkable elements, wherein there is an asymmetry,
**Figure 9** is an ultrasound image comprising two measurement remarkable elements analyzed for ventricles asymmetry first criterion and the remarkable scores of the remarkable elements, wherein there is no asymmetry,
**Figure 10** is an ultrasound image comprising two measurement remarkable elements analyzed for ventricles asymmetry first criterion and the remarkable scores of the remarkable elements, wherein one remarkable element is unobservable,
**Figure 11** is an ultrasound image comprising two measurement remarkable elements analyzed for ventricles asymmetry first criterion and the remarkable scores of the remarkable elements, wherein there is a bigger asymmetry than in figure 8,
**Figure 12** is a representation of three mutually exclusive first scores outputted by the transformation module in relation with the ultrasound image from figure 2, respectively a presence score, an absence score and an assessability score,
**Figure 13** is a representation of three mutually exclusive first scores outputted by the transformation module in relation with the ultrasound image from figure 3, respectively a presence score, an absence score and an assessability score,
**Figure 14** is a representation of three mutually exclusive first scores outputted by the transformation module in relation with the ultrasound image from figure 4, respectively a presence score, an absence score and an assessability score,
**Figure 15** is a representation of a second embodiment of two first scores outputted by the transformation module in relation with the ultrasound image from figure 5, respectively an assessability score and a defect score,
**Figure 16** is a representation of a second embodiment of two first scores outputted by the transformation module in relation with the ultrasound image from figure 6, respectively an assessability score and a defect score,
**Figure 17** is a representation of a second embodiment of two first scores outputted by the transformation module in relation with the ultrasound image from figure 7, respectively an assessability score and a defect score,
**Figure 18** is a representation of three mutually exclusive first scores and a severity score outputted by the transformation module in relation with the ultrasound image from figure 8,
**Figure 19** is a representation of three mutually exclusive first scores and a severity score outputted by the transformation module in relation with the ultrasound image from figure 9,
**Figure 20** is a representation of three mutually exclusive first scores and a severity score outputted by the transformation module in relation with the ultrasound image from figure 10,
**Figure 21** is a representation of three mutually exclusive first scores and a severity score outputted by the transformation module in relation with the ultrasound image from figure 11,
**Figure 22** is an ultrasound image at a first time point comprising two measurement remarkable elements analyzed for ventricles asymmetry first criterion, the remarkable scores of the remarkable elements, and three mutually exclusive first scores associated with the first criterion wherein an asymmetry is observed,
**Figure 23** is an ultrasound image at a second time point comprising two measurement remarkable elements analyzed for ventricles asymmetry first criterion, the remarkable scores of the remarkable elements, and three mutually exclusive first scores associated with the first criterion wherein an asymmetry is observed,
**Figure 24** is an ultrasound image at a third time point comprising two measurement remarkable elements analyzed for ventricles asymmetry first criterion, the remarkable scores of the remarkable elements, and three mutually exclusive first scores associated with the first criterion wherein an asymmetry is observed,
**Figure 25** is an ultrasound image at a first time point comprising two measurement performed on remarkable elements analyzed for ventricles asymmetry first criterion, the remarkable scores of the remarkable elements, and three mutually exclusive first scores associated with the first criterion wherein no asymmetry is observed,
**Figure 26** is an ultrasound image at a second time point comprising two measurement performed on remarkable elements analyzed for ventricles asymmetry first criterion, the remarkable scores of the remarkable elements, and three mutually exclusive first scores associated with the first criterion wherein an asymmetry is observed,
**Figure 27** is an ultrasound image at a third time point comprising two measurement performed on remarkable elements analyzed for ventricles asymmetry criterion, the remarkable scores of the remarkable elements, and three mutually exclusive first scores associated with the first criterion wherein no asymmetry is observed,
**Figure 28** is an ultrasound image at a first time point comprising two measurement performed on remarkable elements analyzed for ventricles asymmetry first criterion, the remarkable scores of the remarkable elements, and three mutually exclusive first scores associated with the first criterion wherein the right ventricle is unobservable,
**Figure 29** is an ultrasound image at a second time point comprising two measurement performed on remarkable elements analyzed for ventricles asymmetry first criterion, the remarkable scores of the remarkable elements, and three mutually exclusive first scores associated with the first criterion wherein the right ventricle is unobservable,
**Figure 30** is an ultrasound image at a third time point comprising two measurement performed on remarkable elements analyzed for ventricles asymmetry first criterion, the remarkable scores of the remarkable elements, and three mutually exclusive first scores associated with the first criterion wherein the right ventricle is unobservable,
**Figure 31** is an ultrasound image at a first time point comprising one remarkable element analyzed for static mitral valve global criterion, wherein the valve is observable and closed,
**Figure 32** is an ultrasound image at a second time point comprising one remarkable element analyzed for static mitral valve global criterion, wherein the valve is observable and closed,
**Figure 33** is an ultrasound image at a third time point comprising one remarkable element analyzed for static mitral valve global criterion, wherein the valve is observable and closed,
**Figure 34** is an ultrasound image at a first time point comprising one remarkable element analyzed for static mitral valve global criterion, wherein the valve is observable and closed,
**Figure 35** is an ultrasound image at a first time point comprising one remarkable element analyzed for static mitral valve global criterion, wherein the valve is observable and openened,
**Figure 36** is an ultrasound image at a first time point comprising one remarkable element analyzed for static mitral valve global criterion, wherein the valve is observable and closed,
**Figure 37** is a representation of three mutually exclusive global scores outputted by the temporal module in relation with the ultrasound images from figures 22 to 24,
**Figure 38** is a representation of three mutually exclusive global scores outputted by the temporal module in relation with the ultrasound images from figures 25 to 27,
**Figure 39** is a representation of three mutually exclusive global scores outputted by the temporal module in relation with the ultrasound images from figures 28 to 30,
**Figure 40** is a representation of two global scores outputted by the temporal module in relation with the ultrasound images from figures 31 to 33,
**Figure 41** is a representation of two global scores outputted by the temporal module in relation with the ultrasound images from figures 34 to 36,
**Figure 42** is a representation of a matrix that may be fed as an input to the temporal module according to an embodiment,
**Figure 43** is a representation of a matrix that may be outputted by the temporal module according to an embodiment,
**Figure 44** is a flow chart showing successive steps executed with the device for guiding of figure 1,
**Figure 45** diagrammatically shows an apparatus, comprising at least one graphic processor, integrating the functions of the device for guiding of figure 1, and
**Figure 46** diagrammatically shows an apparatus, comprising only CPU, integrating the functions of the device for guiding of figure 1, according to a specific embodiment.

### DETAILED DESCRIPTION

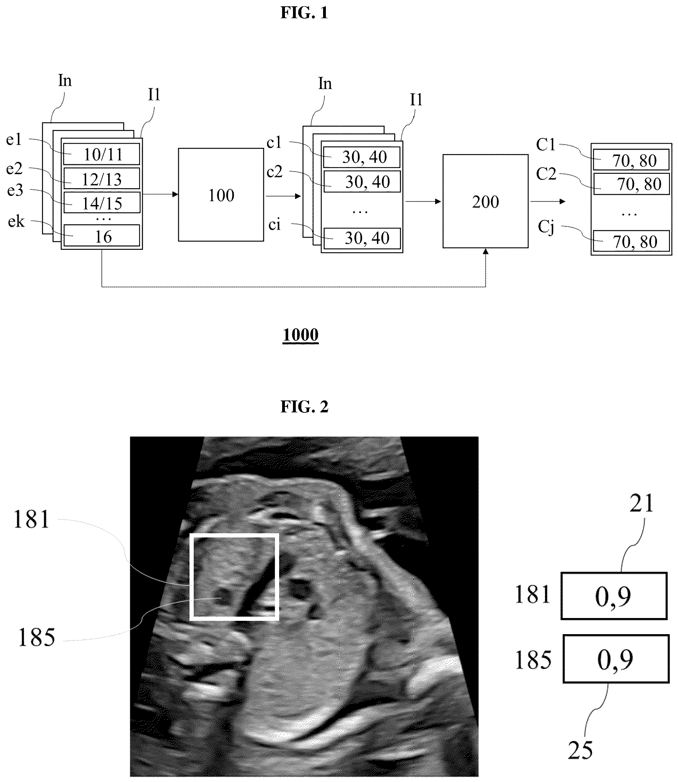

The present disclosure will be described in reference to different embodiments of a device **1000** for guiding a user in ultrasound assessment of an organ, as illustrated on figure 1.

During the years, medical guidelines have defined first criteria **c1**, **c2,...,ci,** that is ultrasound findings indicative of an organ health status that may be used by the physician to diagnose a disease. For instance, a first criterion may be a septal defect, a mass in the left ventricle, or a ventricular asymmetry. Such first criteria **c1**, **c2,...,ci** may be verified based on the analysis of at least one remarkable element **e1**, **e2, e3,...,ek** present in an ultrasound image **I1,...,In.** According to the invention, a remarkable element **e1**, **e2, e3,...,ek** refers to an ultrasound structural feature associated with at least one remarkable information among at least four types of remarkable information. Advantageously, the at least one information associated with each remarkable element **e1**, **e2, e3,...,ek** may be comprised in at least one score.

The device **1000** may comprise a transformation module **100** that receives as input n lists of k remarkable elements **e1**, **e2, e3...,ek.** A list of remarkable elements **e1**, **e2, e3,...,ek** may be extracted from an ultrasound image **I1,...,In** among a set of n ultrasound images **I1,...,In** of the organ. The number of remarkable elements **e1**, **e2, e3,...,ek** that may be extracted may differ depending on the ultrasound image **I1,...,In.** The remarkable elements **e1**, **e2, e3,...,ek** may be extracted based on a predetermined database of remarkable elements **e1**, **e2, e3,...,ek,** for instance using classifiers, such as described in patent applications WO2023031438 and EP20305972.

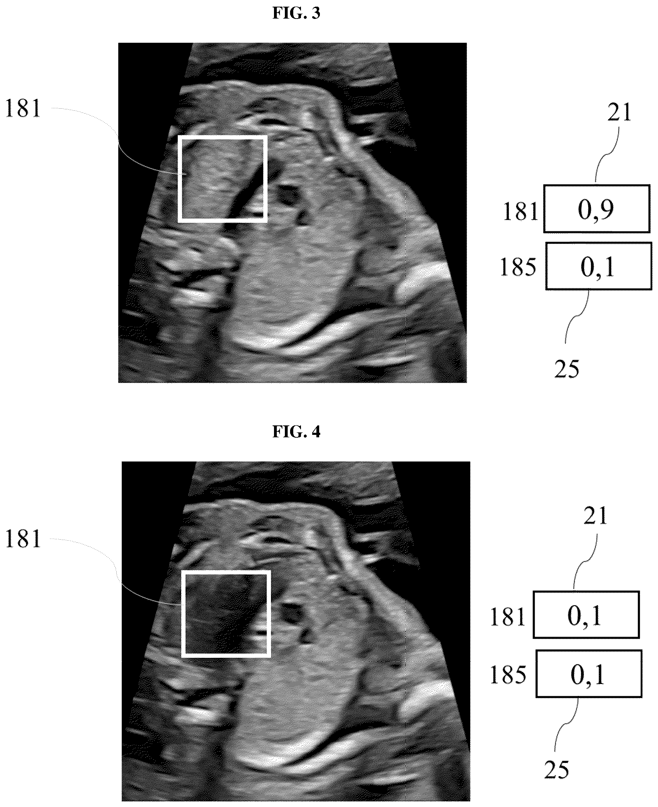

The first type of remarkable information associated to one remarkable element **e1**, **e2, e3,...,ek** relates to an observability **10** or an unobservability **11** of the remarkable element **e1**, **e2, e3...,ek** on a given ultrasound image. The unobservability of the remarkable element may either be caused by a real absence of the remarkable element in the ultrasound image or by an impossibility to detect the remarkable element because of a wrong angle or because of the presence of an artifact ultrasound shadow in the ultrasound image. For instance, in figures 2 to 4, a remarkable element **181** that may be extracted is the region beside the ductal arch. In figures 2 and 3, the region is well observable and the remarkable element **181** can be extracted. However, in figure 4, the region is hidden by a shadow and the remarkable element **181** cannot be correctly extracted because the outlines of the region cannot be determined. Therefore, the remarkable information associated with the remarkable element **181** in figures 2 and 3 is a remarkable information of observability **10.** On the contrary, in figure 4, the remarkable information associated with the remarkable element **181** is a remarkable information of unobservability **11.** To convey the remarkable information **10, 11,** it is possible to provide a remarkable score **21** as an input to the transformation module **100.** Such remarkable score **21** may be comprised between 0 and 1, a remarkable score approaching 0 conveying an unobservability **11** and a remarkable score approaching 1 conveying an observability **10.** For instance, in figures 2 and 3, the remarkable score **21** is equal to 0.9. On the contrary, in figure 4, the remarkable score **21** is equal to 0.1.

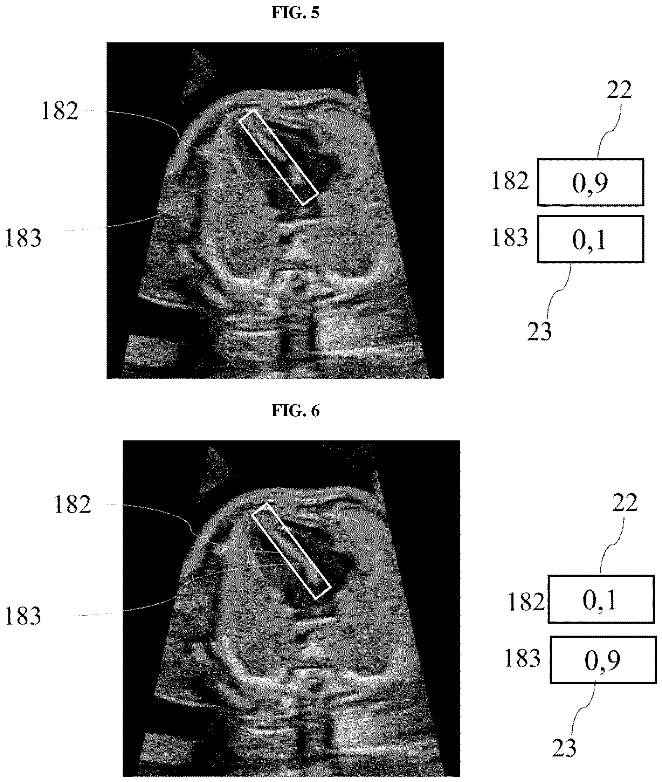

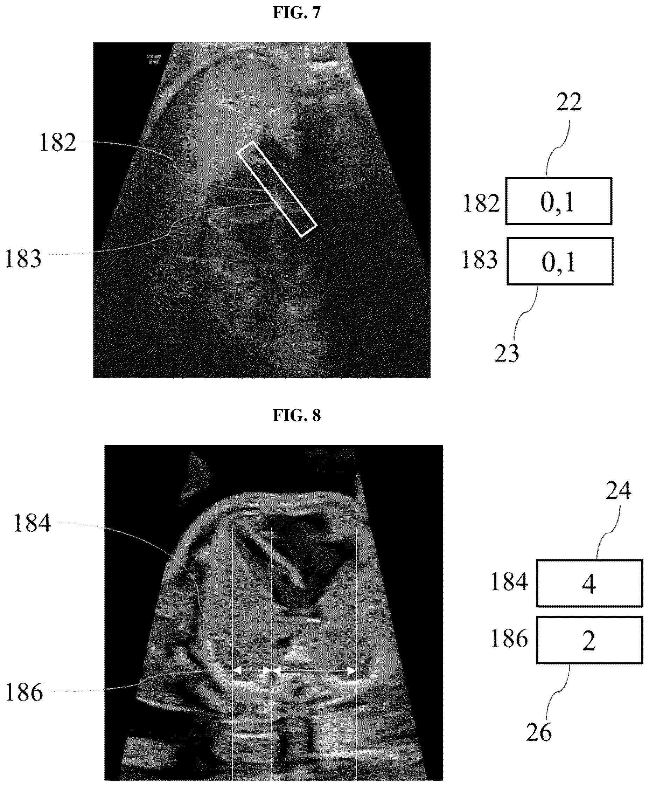

The second type of remarkable information associated to one remarkable element **e1, e2, e3,...,ek** relates to an abnormal aspect **12** of the remarkable element or a non-abnormal aspect **13** of the remarkable element in a given ultrasound image. In that case, the remarkable element is a structural element or feature that is consistently present in abnormal organs. For instance, in figures 5 to 7, a remarkable element **182** that may be extracted is a discontinuous septum. In figure 5, a discontinuous septum can be extracted. However, in figure 6, the septum is continuous, therefore a discontinuous septum cannot be extracted. In figure 7, the septum is unobservable, therefore a discontinuous septum cannot be extracted either. Hence, the remarkable information associated with the remarkable element **182** in figures 5 is an information of abnormal aspect **12.** On the contrary, in figure 6, the remarkable information associated with the remarkable element **182** is a remarkable information of non-abnormal aspect **13.** In figure 7, the remarkable information associated with the remarkable element **182** is also a remarkable information of non-abnormal aspect **13** even though the septum is unobservable. Indeed, the extraction of the remarkable element is binary: either it is detected or not. To convey the remarkable information **12, 13,** it is possible to give a remarkable score **22** as an input to the transformation module **100.** Such remarkable score **22** may be comprised between 0 and 1, a remarkable score approaching 1 conveying an abnormal aspect **12** and a remarkable score approaching 0 conveying a non-abnormal aspect **13.** For instance, in figures 6 and 7, the remarkable score **22** is equal to 0.1. On the contrary, in figure 5, the remarkable score **22** is equal to 0.9.

As another example given in figures 2 to 4, a remarkable element **185** that may be extracted is a vessel present beside the ductal arch. In figure 2, a vessel can be extracted. However, in figure 3, no vessel is present, therefore a vessel cannot be extracted. In figure 4, the region is unobservable, therefore a vessel cannot be extracted either. Hence, the remarkable information associated with the remarkable element **185** in figures 2 is a remarkable information of abnormal aspect **12.** On the contrary, in figure 3, the remarkable information associated with the remarkable element **185** is a remarkable information of non-abnormal aspect **13.** In figure 7, the remarkable information associated with the remarkable element **182** is also a remarkable information of non-abnormal aspect **13.** To convey the remarkable information **12, 13,** a remarkable score **25** is given as an input to the transformation module **100.** Such remarkable score **25** may be comprised between 0 and 1, a remarkable score approaching 1 conveying an abnormal aspect **12** and a remarkable score approaching 0 conveying a non-abnormal aspect **13.** For instance, in figures 3 and 4, the remarkable score **22** is equal to 0.1. On the contrary, in figure 2, the remarkable score **22** is equal to 0.9.

The effective detection of the remarkable element **182** in the ultrasound image may also convey a remarkable information of observability **10.** Indeed, if the remarkable element is detected, it has both an abnormal aspect **12** and it is observable **10.**

The third type of remarkable information associated to one remarkable element **e1, e2, e3,...,ek** relates to a normal aspect **14** of the remarkable element or a non-normal aspect **15** of the remarkable element in a given ultrasound image. In that case, the remarkable element is a structural element or feature that is consistently present in normal organs. For instance, in figures 5 to 7, a remarkable element **183** that may be extracted is a continuous septum. In figure 6, a continuous septum can be extracted. However, in figure 5, the septum is discontinuous, therefore a continuous septum cannot be extracted. In figure 7, the septum is unobservable, therefore a continuous septum cannot be extracted either. Hence, the remarkable information associated with the remarkable element **183** in figure 6 is a remarkable information of normal aspect **14.** On the contrary, in figure 5, the remarkable information associated with the remarkable element **183** is a remarkable information of non-normal aspect **15.** In figure 7, the remarkable information associated with the remarkable element **183** is also a remarkable information of non-normal aspect **15** even though the septum is unobservable. Indeed, the extraction of the remarkable element is binary: either it is detected or not. To convey the remarkable information **14, 15,** it is possible to give a remarkable score **23** as an input to the transformation module **100.** Such remarkable score **23** may be comprised between 0 and 1, a remarkable score approaching 1 conveying a normal aspect **14** and a remarkable score approaching 0 conveying a non-normal aspect **15.** For instance, in figures 5 and 7, the remarkable score **23** is equal to 0.1. On the contrary, in figure 6, the remarkable score **23** is equal to 0.9.

A remarkable element may be associated with several types of information. For instance, the effective detection of the remarkable element **183** in the ultrasound image may also convey a remarkable information of observability **10.** Indeed, if the remarkable element **183** is detected, it has both a normal and aspect **14** and it is observable **10.**

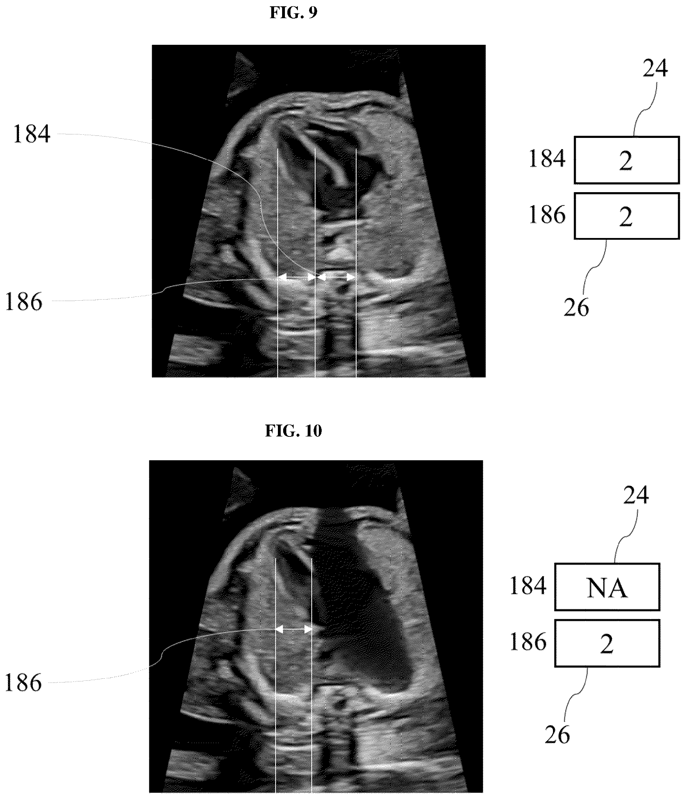

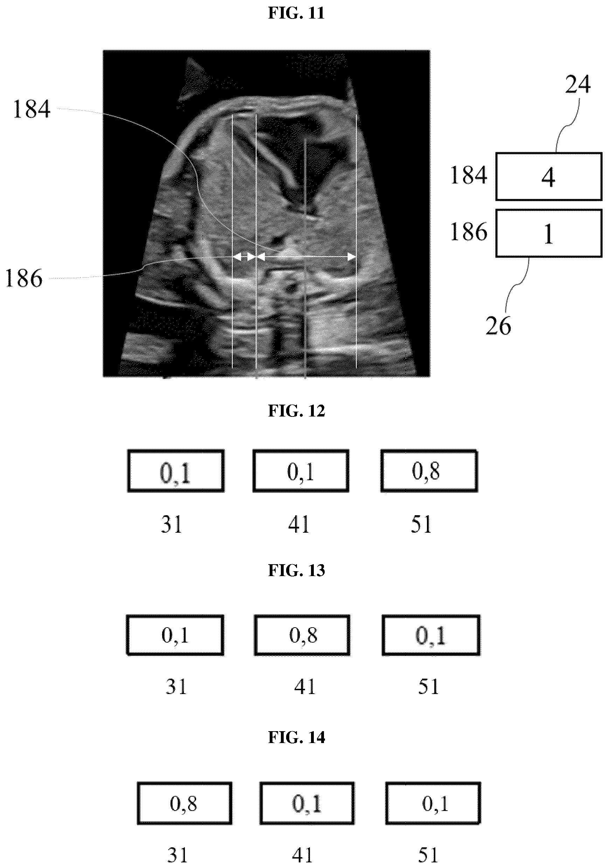

The fourth type of remarkable information associated to one remarkable element **e1**, **e2, e3,...,ek** relates to a measurement **16** performed on the remarkable element such as the measurement of a breadth or an orientation of the remarkable element. For instance, in figures 8 to 11, two remarkable elements **184** and **186** may be extracted: the breadth of the left ventricle and the breadth of the right ventricle. These measurements **16** may be provided as an input to the transformation module **100** and may be used to verify a criterion such as the symmetry between the left and right ventricles. For instance, in figure 8, the breadth of the left ventricle **184** is equal to 1 and the breadth of the right ventricle **186** is equal to 4. In figure 11, the breadth of the left ventricle **184** is equal to 2 and the breadth of the right ventricle **186** is equal to 4. In figure 9, the breadth of the left ventricle **184** is equal to 2 and the breadth of the right ventricle **186** is equal to 2. In figure 10, the breadth of the left ventricle **184** is equal to 2 and the breadth of the right ventricle **186** is non determinable and a NA value is given as an input to the transformation module **100.** Other types of information may be associated to a remarkable element **e1**, **e2, e3,...,ek** such as coordinates.

As illustrated in figure 1, the transformation module **100** is configured, for each criterion of a list of first criteria **c1**, **c2,...,ci,** to convert the remarkable information present in at least one remarkable elements **e1**, **e2, e3,...,ek** into at least two first scores **30, 40.** Each first score **30, 40** conveys at least one first information among a presence of a defect, an absence of a defect and an impossibility to assess the criterion. The first score **30, 40** may be binary scores, thus conveying either that "there is a defect", "there is no defect" or "it is impossible to assess the criterion", and the like. The scores may also be probabilities, thus conveying a probability of presence of a defect, a probability of impossibility to assess the criterion and/or a probability of absence of a defect.

The first scores **30, 40** may be of different types as long as the outputted first scores **30, 40** convey the three types of first information when combined.

The presence of a defect may be determined based on an observability and an abnormal aspect or a non-normal aspect of at least one remarkable element. Alternatively, it may be determined based on an observability of at least one first remarkable element, and an abnormal aspect or a non-normal aspect of at least one second remarkable element. It may also be determined based on an abnormal aspect of at least one first remarkable element and a non-normal aspect of at least one second remarkable element. The absence of a defect information may be determined based on an observability of at least one first remarkable element and a non-abnormal aspect of at least one second remarkable element. It may also be determined based on a non-abnormal aspect of at least one first remarkable element and a normal aspect of at least one second remarkable element. Alternatively, it may be determined based on a normal aspect or a non-abnormal aspect of at least one remarkable element. The presence or the absence of a defect information may be determined based on a comparison of a measurement (i.e., fourth type of information) performed on a first remarkable element with respect to a reference measurement or with a measurement performed on a second remarkable element with respect to a second reference measurement. The impossibility to assess a criterion information may be determined based on an unobservability of at least one remarkable element. The impossibility to assess a criterion information may also be determined based on an unobservability of at least one first remarkable element and a non-abnormal aspect of at least one second remarkable element. Alternatively, the impossibility to assess a criterion may be determined based on a non-abnormal aspect of at least one first remarkable element and a non-normal aspect of at least one second remarkable element.

For instance, a criterion **c1**, **c2,...,ci** may be the fourth vessel beside the ductal arch. Such criterion **c1**, **c2,...,ci** may be evaluated based on two remarkable elements. The first remarkable element is the remarkable element **181** that relates to the observability **10** or unobservability **11** of the region beside the ductal arch. The second remarkable element is the remarkable element **185** that relates to the abnormal presence **12** or non-abnormal presence **13** of a vessel in the region beside the ductal arch.

By evaluating the remarkable information associated with the two remarkable elements **181, 185,** it is possible to conclude on the three first information related to the criterion **c1**, **c2,...,ci.** For instance, in a given ultrasound image **I1,...In,** if the remarkable element **181** gives a remarkable information of observability **10** of the region beside the ductal arch and the remarkable element **185** gives a remarkable information of abnormal aspect **12** related to the presence of a vessel in the region beside the ductal arch, it is possible to conclude to a presence of a defect in the given ultrasound image **I1,...In.**

Alternatively, if the remarkable element **181** gives a remarkable information of observability **10** of the region beside the ductal arch and the remarkable element **185** gives a remarkable information of non-abnormal aspect **13** related to the absence of a vessel in the region beside the ductal arch, it is possible to conclude to an absence of a defect in the given ultrasound image **I1,...,In.**

Lastly, if the remarkable element **181** gives a remarkable information of unobservability **10** of the region beside the ductal arch and the remarkable element **185** gives a remarkable information of non-abnormal aspect **13** related to the absence of a vessel in the region beside the ductal arch, it is possible to conclude to an impossibility to assess the criterion **c1**, **c2,...,ci** in the given ultrasound image **I1,...,In.**

As another example, if the criterion **c1**, **c2,...,ci** is a septal defect, it may be evaluated based on two remarkable elements. The first remarkable element is the remarkable element **183** that relates to the normal aspect **14** or non-normal aspect **15** of the septum. The second remarkable element is the remarkable element **182** that relates to the abnormal aspect **12** or non-abnormal aspect **13** of the septum.

If the remarkable element **182** gives a remarkable information of abnormal aspect **12** related to a discontinuous septum and the remarkable element **183** gives a remarkable information of non-normal aspect **15** also related to a non-continuous septum, it is possible to conclude to a presence of a defect in the given ultrasound image **I1,...,In.**

If the remarkable element **182** gives a remarkable information of non-abnormal aspect **13** related to a non-discontinuous septum and the remarkable element **183** gives a remarkable information of normal aspect **14** also related to a continuous septum, it is possible to conclude to an absence of a defect in the given ultrasound image **I1,...,In.**

Lastly, if the remarkable element **182** gives a remarkable information of non-abnormal aspect **13** and the remarkable element **183** gives a remarkable information of non-normal aspect **14,** it is possible to conclude to an impossibility to assess the criterion in the given ultrasound image because the remarkable information are contradictory.

As another example, the criterion **c1**, **c2,...,ci** may be a ventricular asymmetry. It may be evaluated based on two remarkable elements. The first remarkable element **184** relates to the breadth of the right ventricle and the second remarkable element **186** relates to the breadth of the left ventricle. If the first remarkable element **184** is equal to 4 and the second remarkable element **186** is equal to 2, as illustrated in figure 8, it is possible to conclude to the presence of a defect in the given ultrasound image due to a ventricular asymmetry. If the first remarkable element **184** is equal to 2 and the second remarkable element **186** is equal to 2, as illustrated in figure 9, it is possible to conclude to the absence of a defect in the given ultrasound image because both ventricles have the same size. If the first remarkable element **184** is equal to NA and the second remarkable element **186** is equal to 2, as illustrated in figure 10, it is possible to conclude to an impossibility to assess the criterion **c1**, **c2,...,ci** in the given ultrasound image **I1...In** because one ventricle is non observable.

The outputted first scores may take different shapes. For instance, as illustrated in figure 12, three mutually exclusive first scores may be defined: an assessability score **31,** and a presence score **41** and an absence score **51.** The first scores **31, 51, 41** may for instance take a value comprised between 0 and 1. For example, for the assessability score **31,** a value approaching 0 may mean that the criterion is assessable. On the contrary, a value approaching 1 means that the criterion **c1**, **c2,...,ci** is impossible to assess. For the presence score **41,** a value approaching 1 means a high probability of presence of a defect. For the absence score **51,** a value approaching 1 means a high probability of absence of a defect. The sum of the three first scores **31, 51, 41** may be equal to 1, conveying the fact that the criterion cannot correspond both to a presence of a defect and an absence of a defect or correspond both to an absence of a defect and be impossible to assess or correspond both to a presence of a defect and be impossible to assess.

Figures 12 to 14 shows first scores **31, 51, 41** that may be obtained by the transformation module **100** based on the ultrasound image from figure 2 to 4. As explained before, two remarkable elements **181, 185** may be extracted from figure 2 to 4. A first remarkable element **181** that corresponds to the observability of the region beside the ductal arch and a second remarkable element **185** that corresponds to a vessel in the region beside the ductal arch.

In figure 2, the remarkable score **21** associated with the first remarkable element **181** is 0.9 and the remarkable score **25** associated with the second remarkable element **185** is 0.9. The first scores **31, 51, 41** outputted by transformation module **100** corresponding to the fourth vessel beside the ductal arch criterion are illustrated in figure 12. The assessability score **31** is equal to 0.1 meaning that the criterion is assessable, the presence score **41** is equal to 0.8, meaning that the probability of presence of a defect is high and the absence score **51** is equal to 0.1, meaning that the probability of absence of a defect is low. The sum of the three first scores **31, 51, 41** is equal to 0.1 + 0.1+0.8 = 1.

In figure 3, the remarkable score **21** associated with the first remarkable element **181** is 0.9 and the remarkable score **22** associated with the second remarkable element **185** is 0.1. The first scores **31, 51, 41** outputted by transformation module **100** corresponding to the fourth vessel beside the ductal arch criterion are illustrated in figure 13. The assessability score **31** is equal to 0.1 meaning that the criterion is assessable, the presence score **41** is equal to 0.1, meaning that the probability of presence of a defect is low and the absence score **51** is equal to 0.8, meaning that the probability of absence of a defect is high. The sum of the three first scores is equal to 0.1 + 0.8+0.1 = 1.

In figure 4, the remarkable score **21** associated with the first remarkable element **181** is 0.1 and the remarkable score **25** associated with the second remarkable element **185** is 0.1. The first scores **31, 51, 41** outputted by transformation module **100** corresponding to the fourth vessel beside the ductal arch criterion are illustrated in figure 14. The assessability score **31** is equal to 0.8 meaning that the criterion is not assessable, the presence score **41** is equal to 0.1 and the absence score **51** is equal to 0.1. Therefore, the probability of presence and absence of a defect are both low because the transformation module **100** is unable to draw a conclusion on this specific ultrasound image. The sum of the three first scores **31, 51, 41** is equal to 0.8 + 0.1+0.1 = 1.

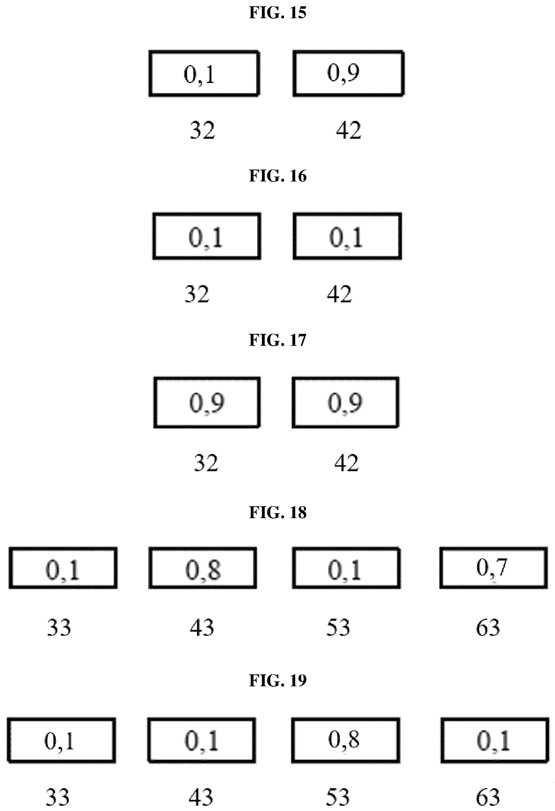

Alternatively, as illustrated in figure 15, two first scores **32, 42** may be defined: a defect score **42** including the presence of a defect and the absence of a defect and an assessability score **32,** including the impossibility to assess the criterion. The first scores **32, 42** may for instance take a value comprised between 0 and 1. For example, for the assessability score **32,** a value approaching 0 may mean that the criterion cannot be correctly assessed. On the contrary, a value approaching 1 means that the criterion can be correctly assessed. Alternatively, a value approaching 0 may convey a possibility to assess the criterion and a value approaching 1 may convey an impossibility to assess the criterion. For the defect score **42,** a value approaching 0 means a high probability of presence of a defect with respect to the criterion, while a value approaching 1 means a high probability of absence of a defect with respect to the criterion. Alternatively, a value approaching 0 may mean a high probability of absence of a defect a value approaching 1 means a high probability of presence of a defect.

Figures 15 to 17 show first scores **32, 42** that may be obtained by the transformation module **100** based on the ultrasound image from figure 5 to 7. As explained before, two remarkable elements **182, 183** may be extracted from figure 5 to 7. A first remarkable element **182** that corresponds to a discontinuous septum and a second remarkable element **183** that corresponds to a continuous septum.

In figure 5, the remarkable score **22** associated with the first remarkable element **182** is 0.9 and the remarkable score **23** associated with the second remarkable element **183** is 0.1. The first scores **32, 42** outputted by transformation module **100** corresponding to the septal defect criterion are illustrated in figure 15. The assessability score **32** is equal to 0.1 meaning that the criterion is assessable, the defect score **42** is equal to 0.9, meaning that the probability of presence of a defect is high.

In figure 6, the remarkable score **22** associated with the first remarkable element **182** is 0.1 and the remarkable score **23** associated with the second remarkable element **183** is 0.9. The first scores **32, 42** outputted by transformation module **100** corresponding to the septal defect criterion are illustrated in figure 16. The assessability score **32** is equal to 0.1 meaning that the criterion is assessable, the defect score **42** is equal to 0.1, meaning that the probability of absence of a defect is high.

In figure 7, the remarkable score **22** associated with the first remarkable element **182** is 0.1 and the remarkable score **23** associated with the second remarkable element **183** is 0.1. The first scores **32, 42** outputted by transformation module **100** corresponding to the septal defect criterion are illustrated in figure 17. The assessability score **32** is equal to 0.9 meaning that the criterion is not assessable, the defect score **42** is equal to 0.9 but this value should be interpreted as meaningless to the physician.

Advantageously, a severity score may be included. The severity score may be comprised between 0 and 1 wherein 0 means a low severity and 1 means a high severity of the defect. With reference to figures 18 to 21, it possible to define 4 scores: an assessability score **33,** a presence score **43,** an absence score **53** and a severity score **63.**

Figure 18 to 21 shows first scores **33, 43, 53, 63** that may be obtained by the transformation module **100** based on the ultrasound imaged from figure 8 to 11. As explained before, two remarkable elements **184, 186** may be extracted from figure 8 to 11. A first remarkable element **184** that corresponds to the breadth of the right ventricle and a second remarkable element **183** that corresponds to the breadth of the left ventricle.

In figure 8, the value **24** associated with the first remarkable element **184** is 4 and the value **26** associated with the second remarkable element **186** is 2. The first scores **33, 43, 53, 63** outputted by transformation module **100** corresponding to the ventricular asymmetry are illustrated in figure 18. The assessability score **33** is equal to 0.1 meaning that the criterion is assessable, the presence score **43** is equal to 0.8, meaning that the probability of presence of a defect is high because the ventricles don't have the same breadth in this ultrasound image, the absence score **53** is equal to 0.1, meaning that the probability of absence of a defect is low and the severity score **63** is equal to 0.7, meaning that the asymmetry is quite severe.

In figure 9, the value **24** associated with the first remarkable element **184** is 2 and the value **26** associated with the second remarkable element **186** is 2. The first scores **33, 43, 53, 63** outputted by transformation module **100** corresponding to the ventricular asymmetry are illustrated in figure 19. The assessability score **33** is equal to 0.1 meaning that the criterion is assessable, the presence score **43** is equal to 0.1, meaning that the probability of presence of a defect is low, the absence score **53** is equal to 0.8, meaning that the probability of absence of a defect is high because both ventricles have the same breadth in this ultrasound image and the severity score **63** is equal to 0.1, meaning that there is no asymmetry.

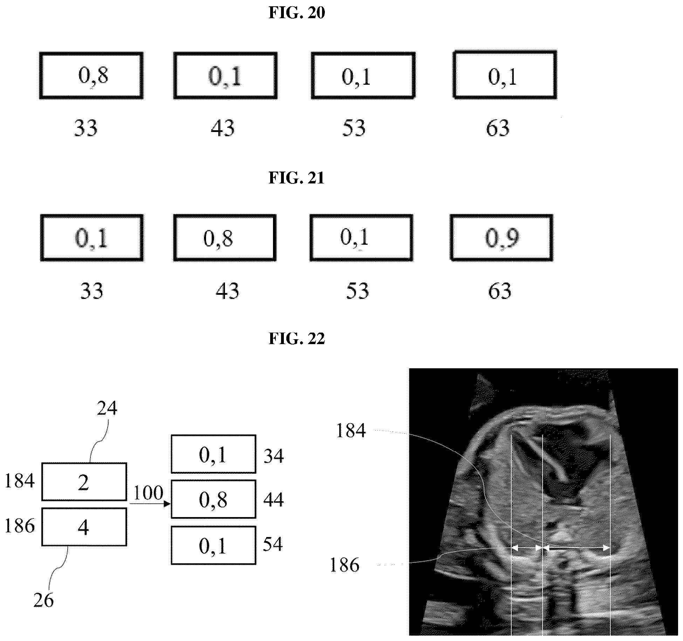

In figure 10, the value **24** associated with the first remarkable element **184** is NA and the value **26** associated with the second remarkable element **186** is 2. The first scores **33, 43, 53, 63** outputted by transformation module **100** corresponding to the ventricular asymmetry are illustrated in figure 20. The assessability score **33** is equal to 0.8 meaning that the criterion is impossible to assess, the presence score **43,** absence score **53** and severity score **63** are equal to 0.1.

In figure 11, the value **24** associated with the first remarkable element **184** is 4 and the value **26** associated with the second remarkable element **186** is 1. The first scores **33, 43, 53, 63** outputted by transformation module **100** corresponding to the ventricular asymmetry are illustrated in figure 21. The assessability score **33** is equal to 0.1 meaning that the criterion is assessable, the presence score **43** is equal to 0.8, meaning that the probability of presence of a defect is high because there is an asymmetry between the ventricles, the absence score **53** is equal to 0.1, meaning that the probability of absence of a defect is low. The severity score **63** is equal to 0.9 in this ultrasound image. Indeed, compared to figure 8, the asymmetry is much more severe than in figure 8 because the difference between the ventricles breadth is bigger in figure 11 compared to figure 8.

The first scores may be obtained using arithmetic operators of linear or non-linear nature such as a CNN or arithmetic means, weighted means, geometric means or harmonic means.

For instance, the transformation module **100** may be configured to take as input a matrix with two dimensions 2x3. The 2 lines correspond to two remarkable elements. The first column corresponds to a remarkable score associated to each remarkable element. The remarkable score comprises observability/unobservability information. The remarkable score is comprised between 0 and 1, and the last two columns correspond to location information in the form of coordinates of the remarkable elements.

For instance, the transformation module **100** may be configured to output a matrix with two dimensions 1x2. The line corresponds to a first criterion and the two columns to two first scores. One first score may be extracted as the mean of the observability scores of the two remarkable elements. The second first score may be extracted as the division between the two breadths measurement calculated from the coordinates of the remarkable elements, followed by a normalization operation to obtain a value equal to 0 if the ratio is close to reference value, and value equal to 1 if the ratio is far from reference value, power the mean of the observability scores.

As another example, the transformation module **100** may be configured to output a matrix with two dimensions 1x3. The line corresponds to a first criterion and the three columns to three remarkable scores comprising respectively observability/unobservability, normality/non-normality and abnormality/non-abnormality information. The first scores can be extracted through a multi-class softmax classification which outputs three mutually exclusive classification confidences.

Because the ultrasound images are only partial views of the organ, the outputted first scores may be biased, for instance because of the ultrasound image angle. The temporal module **200** is configured to aggregate the information extracted from all the ultrasound images **I1,...In,** to output global information representative of the actual and unbiased health status of the organ.

As illustrated in figure 1, the lists of remarkable elements **e1**, **e2, e3,...,ek** and/or the lists of first criteria **c1**, **c2,...,ci** obtained for each ultrasound image **I1,...In** are fed to a temporal module **200.** The temporal module **200** is configured to aggregate the first scores **30, 40** outputted by the transformation module **100** for each image of the set of ultrasound images **I1,...,In** and to output a list of global criteria **C1, C2,...,Cj,** wherein each criterion of the list of global criteria **C1, C2,...,Cj,** is associated with at least two global scores **70, 80** corresponding to the behavior of a corresponding criterion of the list of first criteria **c1**, **c2,...,ci** across the whole set of ultrasound images **I1,...,In.**

The global scores are derived from an analysis of the remarkable elements across at least a sub-set of ultrasound images comprising at least two ultrasound images or, preferably, the whole set of ultrasound images. Indeed, one same remarkable element may or may not be observed in different ultrasound images depending on the viewpoint of the ultrasound image and on the quality of the ultrasound image. If one remarkable element is consistently associated with the same remarkable information, it may indicate a high probability of presence or an absence of a defect. On the other hand, if the remarkable element appears in different locations, presents different dimensions or is difficult to identify in some images, this may either indicate that the set of ultrasound images is not qualitative enough to draw conclusions on the global criterion, or it may indicate that there is a problem with the organ with respect to the global criterion, such as a lesion or an abnormal growth.

For instance, a global criterion may be a global ventricular asymmetry. The global asymmetry may be evaluated over several ultrasound images **I1,...,In.** Indeed, the heart being a dynamic organ, its shape changes periodically through time. Throughout this change, the visual aspect ratio of globally asymmetrical ventricles can seem normal sporadically on some ultrasound images **I1,...,In.** Inversely, the visual aspect ratio of globally symmetrical ventricles can seem abnormally asymmetrical sporadically on other ultrasound images **I1,...,In**. The global scores **70, 80** take into account first scores determined over a large number of images **I1,...,In.** Therefore, they are more representative of the actual ratio between the ventricles.

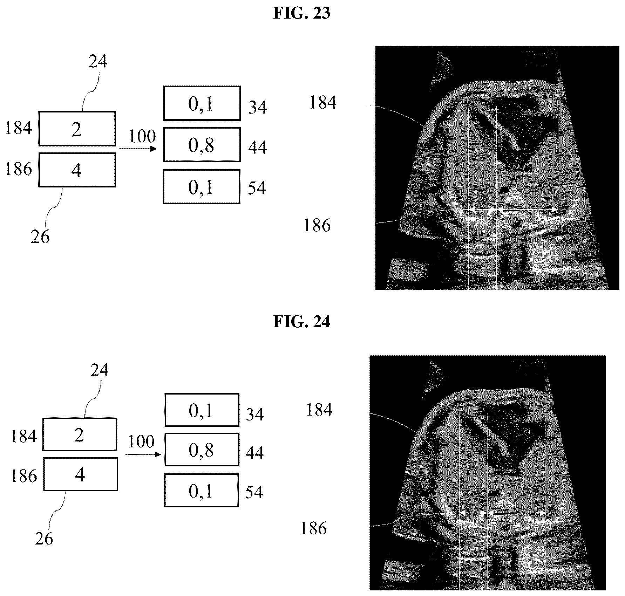

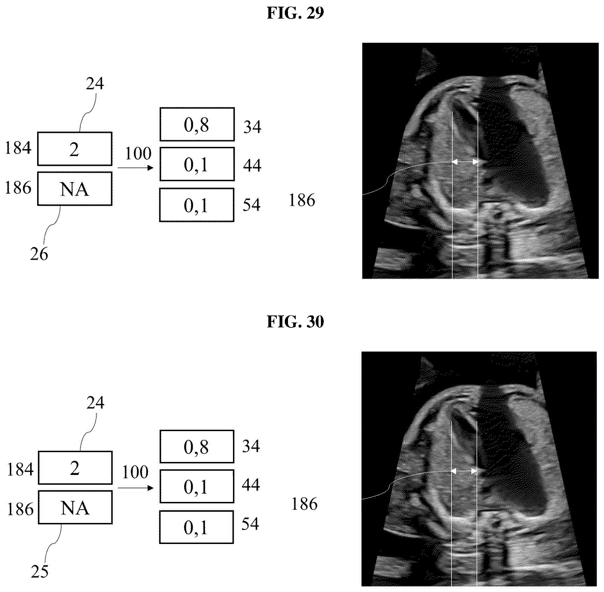

As an example, 3 ultrasound images may be used as illustrated in figures 22 to 24, figures 25 to 27 or figures 28 to 30. In these ultrasound images, remarkable elements **184** and **186** have been extracted. These remarkable elements **184, 186** respectively correspond to the breadth of the right and left ventricles. The transformation module **100** outputted three mutually exclusive first scores **34, 44, 54** corresponding to an assessability score **34,** a presence score **44** and an absence score **54.**

In the three ultrasound images from figures 22 to 24, there is an asymmetry between the ventricles and the presence score **44** is equal to 0.8, while the assessability score **34** and the absence score **54** are equal to 0.1. Therefore, the global scores will also conclude to the global presence of a defect because all ultrasound images contain the same first information of presence of a defect.

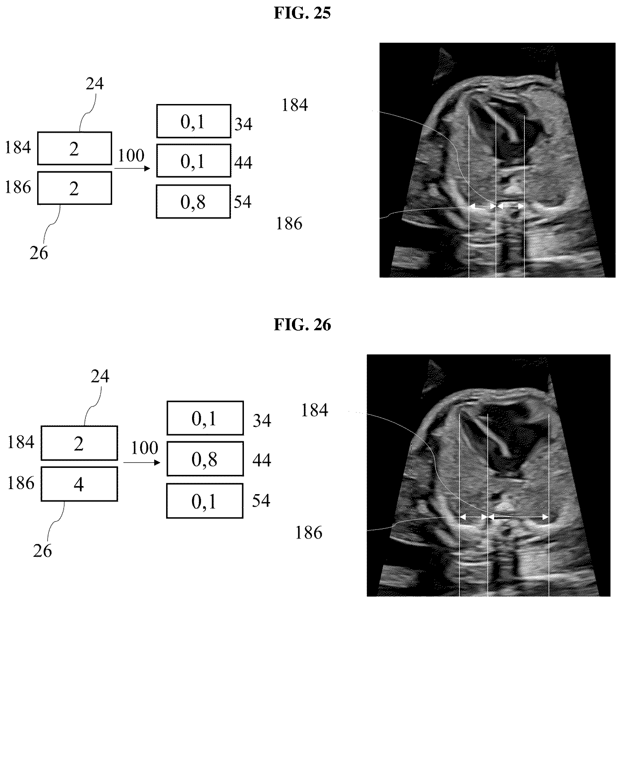

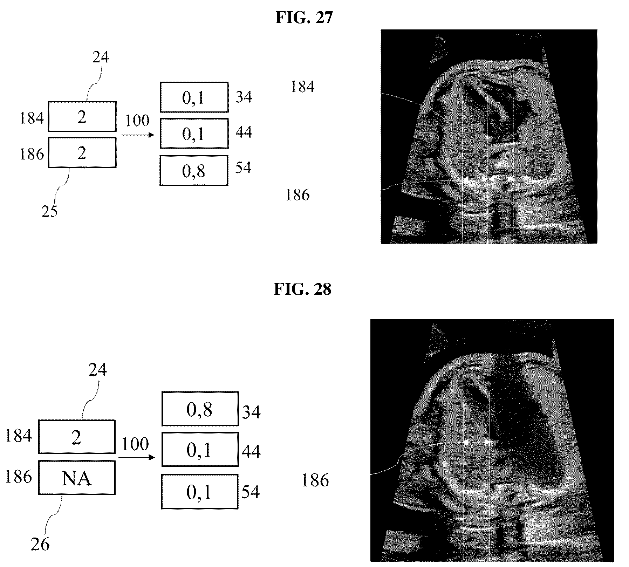

In the three ultrasound images from figures 25 to 27, there is no asymmetry between the ventricles in figures 25 and 27. The absence score **44** is equal to 0.8 in these two figures, while the assessability score **34** and the presence score **54** are equal to 0.1. However, there is an asymmetry in figure 26 and the presence score **44** is equal to 0.8, while the assessability score **34** and the absence score **54** are equal to 0.1. In this case, the global scores will conclude to the global absence of a defect because a majority of the ultrasound images contain the same first information of absence of a defect.

In the three ultrasound images from figures 28 to 30, there is a shadow over the right ventricle and the remarkable element **186** is impossible to measure. Therefore, the assessability score **34** is equal to 0.8, while the presence score **44** and the absence score **54** are equal to 0.1. In this case, the global scores will conclude to the global impossibility to assess the set of ultrasound images because all the ultrasound images contain the same first information of impossibility to assess the criterion.

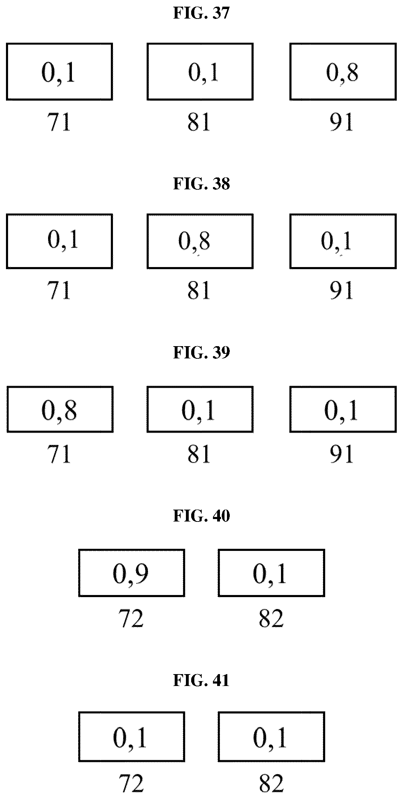

As illustrated in figure 37 to 39, the global scores may correspond to three mutually exclusive global scores **71, 81, 91** : a global assessability score **71,** a global absence score **81** and a global presence score **91.** The global scores **71, 81, 91** may for instance take a value comprised between 0 and 1. For example, for the global assessability score **71,** a value approaching 0 may mean that the global criterion is assessable. On the contrary, a value approaching 1 means that the global criterion **C1, C2,...,Cj** is impossible to assess. For the absence score **81,** a value approaching 1 means a global high probability of absence of a defect. For the presence aspect score **91,** a value approaching 1 means a global high probability of presence of a defect. The sum of the three global scores **71, 81, 91** may be equal to 1, conveying the fact that the global criterion cannot correspond both to a global presence of a defect and a global absence of a defect or correspond both to a global absence of a defect and be impossible to assess or correspond both to a global presence of a defect and be impossible to assess.

The global scores that may be obtained using the temporal module **200** based on the ultrasound images from figures 22 to 24 are illustrated in figure 37. The global assessability score **71** is equal to 0.1 meaning that the global criterion is assessable, the global absence score **81** is equal to 0.1, meaning that the global probability of absence of a defect is low and the global presence score **91** is equal to 0.8, meaning that the global probability of presence of a defect is high. The sum of the three global scores **71, 81, 91** is equal to 0.1 + 0.1+0.8 = 1.

The global scores that may be obtained using the temporal module **200** based on the ultrasound images from figures 25 to 27 are illustrated in figure 38. The global assessability score **71** is equal to 0.1 meaning that the global criterion is assessable, the global absence score **81** is equal to 0.8, meaning that the global probability of absence of a defect is high and the global presence score **91** is equal to 0.1, meaning that the global probability of presence of a defect is low. The sum of the three global scores **71, 81, 91** is equal to 0.1 + 0.8+0.1 = 1.

The global scores that may be obtained using the temporal module **200** based on the ultrasound images from figures 28 to 30 are illustrated in figure 39. The global assessability score **71** is equal to 0.8 meaning that the global criterion is impossible to assess, the global absence score **81** and the global presence score 9**1** are equal to 0.1. The sum of the three global scores **71, 81, 91** is equal to 0.8 + 0.1+0.1 = 1.

Alternatively, two global scores may be defined: a global defect score **72** including the global presence of a defect and the global absence of a defect, and a global assessability score **82,** including the global impossibility to assess the global criterion. The global scores **72, 82** may for instance take a value comprised between 0 and 1. For example, for the global assessability score **82,** a value approaching 0 may mean that it is impossible to assess the global criterion. On the contrary, a value approaching 1 means that it is possible to assess the global criterion. For the global defect score **72,** a value approaching 0 means a high probability of global presence of a defect, while a value approaching 1 means a high probability of global absence of a defect.

For instance, a global criterion **C1, C2,...,Cj** may be a static mitral valve.

In that specific case, the criterion cannot be assessed using a single ultrasound image because it is a temporal criterion. Indeed, a valve being a dynamic element, it periodically opens and closes through time. Therefore, it is meaningless to try to determine first criterion **c1**, **c2,...,ci** scores on the mitral valve using the transformation module **100** as some ultrasound images may show the valve closed and some other images may show the valve opened. The temporal module **200** is configured to aggregate these findings to verify if the valve is regularly opening or closing. In that case, the temporal module directly takes the remarkable information associated with at least one remarkable element as an input.

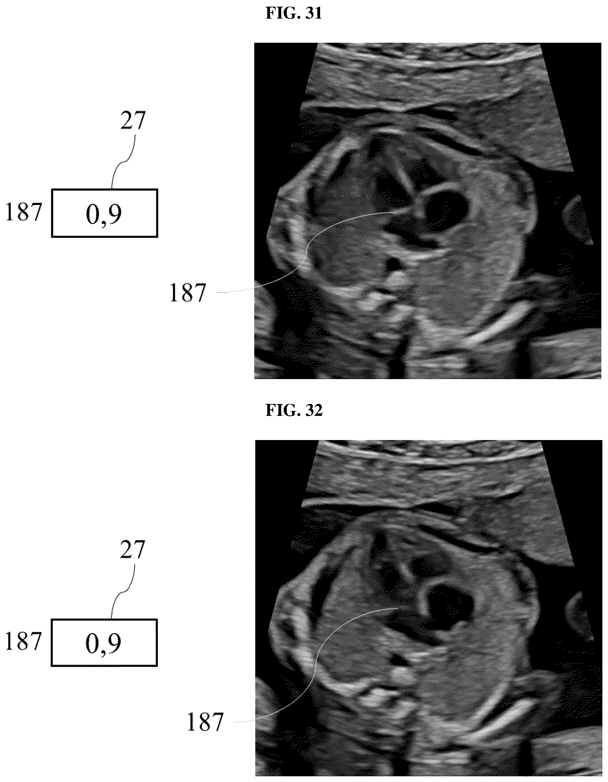

Accordingly, this global criterion **C1, C2,...,Cj** may be assessed directly using remarkable element **187** as illustrated in figures 31 to 33 and 34 to 36. Alternatively, the remarkable information contained in the remarkable element **187** may be stored by the transformation module **100** in the list of first criteria **c1**, **c2,...,ci** of the ultrasound image but the value will be meaningless to the physician as it does not correspond to a score.

In figures 31 to 33, the static mitral valve is always closed. The remarkable element **187** is associated with a remarkable score **27** comprised between 0 and 1, wherein a value approaching 0 means that the mitral valve is well observable and is in a closed state, and a value approaching 1 means that the mitral valve is not observable and in an open state. In figures 31 to 33, the remarkable score **27** is always equal to 0.9.

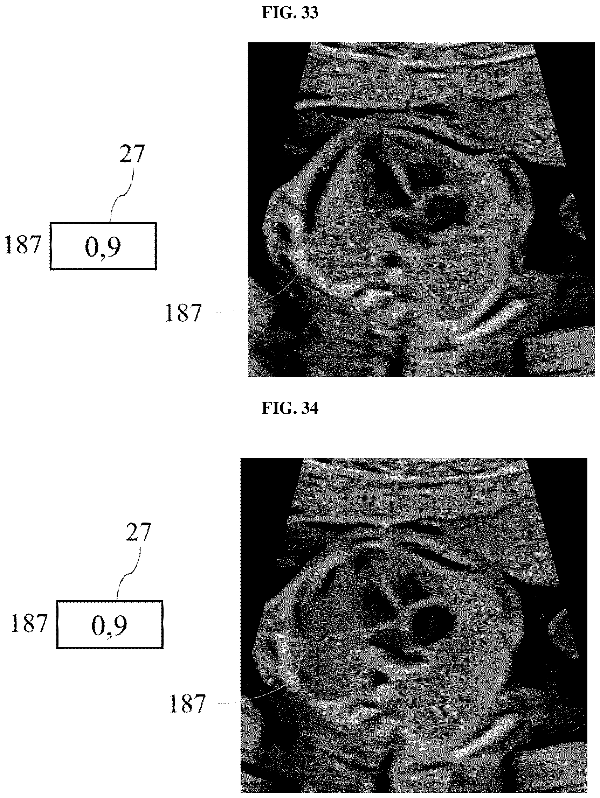

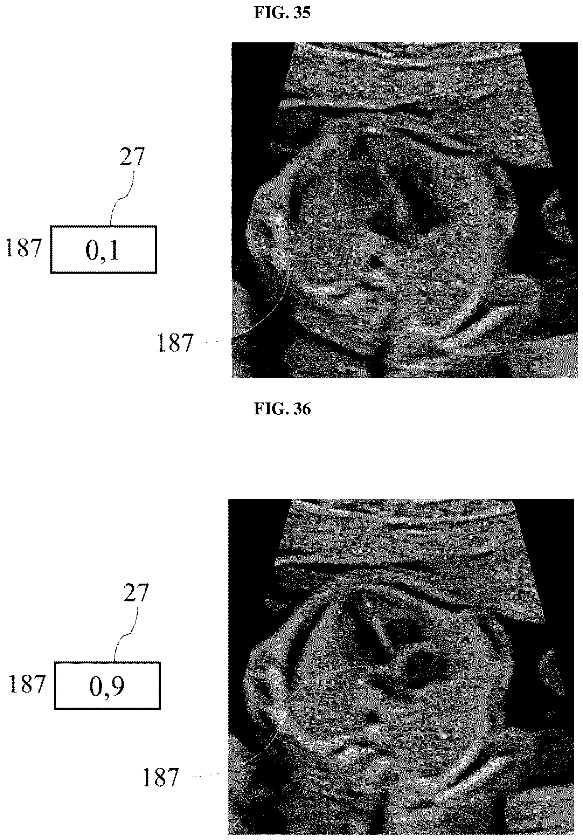

In figures 34 to 36 the static mitral valve is periodically opening and closing. As a consequence, the remarkable score **27** is equal to 0.9 in figures 34 and 36 and the remarkable score **27** is equal to 0.1 in figures 35.

The two global scores **72, 82** associated with figures 31 to 33 are illustrated in figure 40. The global defect score **72** is equal to 0.9 and the global assessability score **82** is equal to 0.1, thus conveying that the static mitral valve is well observable but always closed.

The two global scores **72, 82** associated with figures 34 to 36 are illustrated in figure 41. The global defect score **72** is equal to 0.1 and the global assessability score **82** is equal to 0.1, thus conveying that the static mitral valve is well observable and periodically opening and closing.

To output the global scores, the temporal module **200** implements a feed-forward network of arithmetic operators of linear or non-linear nature such as a 1-dimensional CNN. Such CNN processes simultaneously the lists of first criteria **c1**, **c2,...,ci** outputted by the transformation module **100** to outputs the list of global criteria. Alternatively, the temporal module **200** may be configured to implement a recurrent CNN configured to iteratively process each list of first criteria **c1**, **c2,...,ci** to output the list of global criteria **C1, C2,...,Cj.**

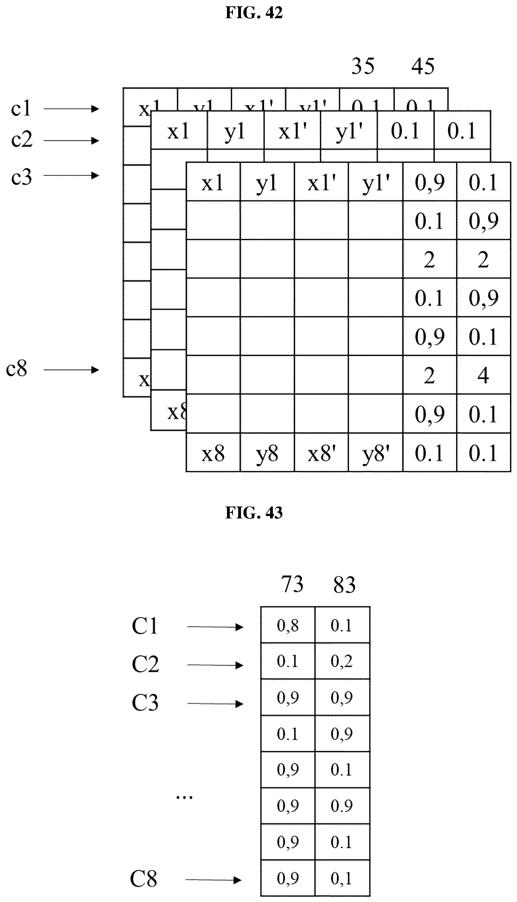

For instance, the CNN can be configured to take as input a matrix with three dimensions 6x8x50 such as illustrated in figure 42. The 8 lines correspond to the first criteria **c1-c8.** The first four column correspond to location information in the form of coordinates **x1,...,x8, y1,...,y8, x1',...x8', y1',...y8'** of a bonding box corresponding to the position in the ultrasound image of the remarkable elements used to verify the criterion. The fifth and sixth columns corresponds to the at least two first scores **35, 45** determined by the transformation module **100.** The third dimension corresponds to the number of ultrasound image **I1,** ..., **In,** which in this case is equal to 50.

One or more convolutional layers may be successively applied to this matrix. The layers are defined by N kernels of size T followed by a non-linear activation function.

The CNN may output a matrix with two dimensions 2x8 such as illustrated in figure 43. The 8 lines correspond to the global criteria **C1-C8.** The two columns correspond to the at least two global scores **73, 83.**

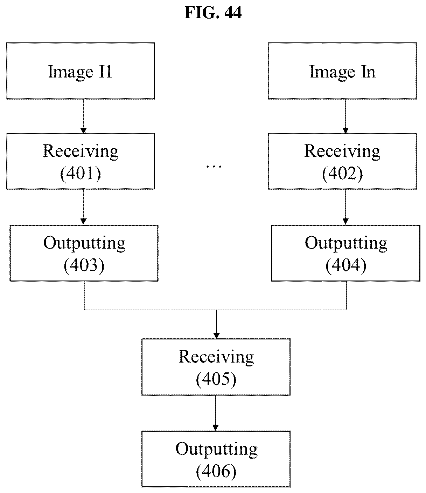

According to another aspect, the invention relates to a method implemented by the device **1000,** as illustrated on figure 44. For each ultrasound image of a set of ultrasound images **I1,...,In,** the method carries out step **401, 402** of receiving a list of remarkable elements and step **403, 404** of outputting a list of first criteria with at least two first scores per criterion of the list of first criteria. These steps may be carried out successively or simultaneously. Once all the lists of first criteria have been generated for all the ultrasound images, the method carries out step **405** of receiving all the outputted lists of first criteria and step **406** of outputting a list of global criteria with at least two global scores per criterion of the list of global criteria.

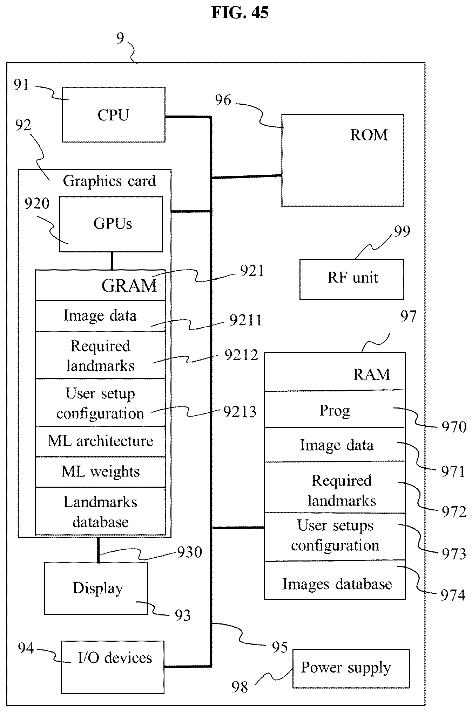

A particular apparatus **9,** visible on figure 45, is embodying the device **1000** for guiding a user in ultrasound assessment of an organ described above. It corresponds, for example, to a workstation, a laptop, a tablet, a smartphone, or a head-mounted display (HMD).

That apparatus **9** is suited for guiding a user in ultrasound assessment of an organ. It comprises the following elements, connected to each other by a bus **95** of addresses and data that also transports a clock signal:
- a microprocessor **91** (or CPU);
- a graphics card **92** comprising several Graphical Processing Units (or GPUs) **920** and a Graphical Random Access Memory (GRAM) **921;** the GPUs are quite suited to image processing by e.g. the CNN block, due to their highly parallel structure;
- a non-volatile memory of ROM type **96;**
- a RAM **97;**
- one or several I/O (Input/Output) devices **94** such as for example a keyboard, a mouse, a trackball, a webcam; other modes for introduction of commands such as for example vocal recognition are also possible;
- a power source **98;** and
- a radiofrequency unit **99.**

According to a variant, the power supply **98** is external to the apparatus **9.**

The apparatus **9** also comprises a display device **93** of display screen type directly connected to the graphics card **92** to display synthesized images calculated and composed in the graphics card. The use of a dedicated bus to connect the display device **93** to the graphics card **92** offers the advantage of having much greater data transmission bitrates and thus reducing the latency time for the displaying of images composed by the graphics card, for example for identifying and localizing the landmarks. According to a variant, a display device is external to apparatus **9** and is connected thereto by a cable or wirelessly for transmitting the display signals. The apparatus **9,** for example through the graphics card **92,** comprises an interface for transmission or connection adapted to transmit a display signal to an external display means such as for example an LCD or plasma screen or a video-projector. In this respect, the RF unit **99** can be used for wireless transmissions.

It is noted that the word "register" used hereinafter in the description of memories **97** and **921** can designate in each of the memories mentioned, a memory zone of low capacity (some binary data) as well as a memory zone of large capacity (enabling a whole program to be stored or all or part of the data representative of data calculated or to be displayed). Also, the registers represented for the RAM **97** and the GRAM **921** can be arranged and constituted in any manner, and each of them does not necessarily correspond to adjacent memory locations and can be distributed otherwise (which covers notably the situation in which one register includes several smaller registers).

When switched-on, the microprocessor **91** loads and executes the instructions of the program contained in the RAM **97.**

As will be understood by a skilled person, the presence of the graphics card **92** is not mandatory, and can be replaced with entire CPU processing and/or simpler visualization implementations.

In variant modes, the apparatus **9** may include only the functionalities of the device **1,** and not the learning capacities of the device for training. In addition, the device **1** and/or the device for training may be implemented differently than a standalone software, and an apparatus or set of apparatus comprising only parts of the apparatus **9** may be exploited through an API call or via a cloud interface.

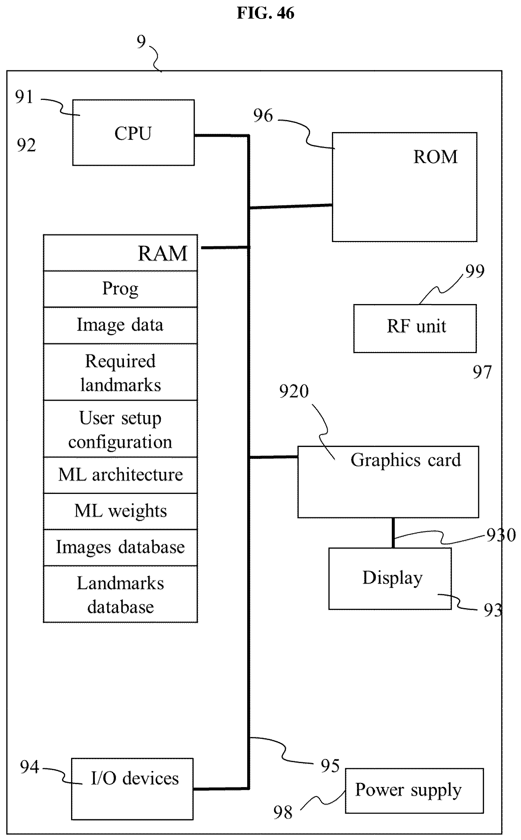

Figure 46 shows an alternative example for the apparatus **9** comprising an appropriate GPU (one that can be used for ML calculation). For example, a simple video card may be integrated into processor. It has no CUDA technology able to paralyze numerical computations.

## Claims

1. A device (1000) for guiding a user in ultrasound assessment of an organ to perform an evaluation of said organ during a medical examination, said ultrasound assessment being based on at least two ultrasound images (I1, ..., In) resulting from at least a partial reflection of ultrasound waves by said organ, said device comprising at least one processor configured to:
• for each ultrasound image (I1, ..., In):
- receive as an input a list of remarkable elements (e1, e2, e3,...,ek, 181-187) associated to the ultrasound image (I1, ..., In), wherein each remarkable element (e1, e2, e3,...,ek, 181-187) is associated with at least one remarkable information among:
o an observability (10) of the remarkable element (e1, e2, e3,...,ek, 181-187) or an unobservability (11) of the remarkable element (181-187),
o an abnormal aspect (12) of the remarkable element (e1, e2, e3,...,ek, 181-187) or a non-abnormal aspect (13) of the remarkable element (e1, e2, e3,...,ek, 181-187),
o a normal aspect (14) of the remarkable element (e1, e2, e3,...,ek, 181-187) or a non-normal aspect (15) of the remarkable element (e1, e2, e3,...,ek, 181-187), and
▪ a measurement performed on the remarkable element (e1, e2, e3,...,ek, 181-187),
- generate and output a list of first criteria (c1, c2, c3-c8,...ci) indicative of a health status of the organ, wherein each first criterion of the list of first criteria (c1, c2, c3-c8,...,ci) is associated with at least two first scores (30-35, 40-45, 41, 53, 54, 63), each first score (30-35, 40-45, 41, 53, 54, 63) being generated using said at least one remarkable information associated to the at least one remarkable element (e1, e2, e3,...,ek, 181-187), said at least two first scores (30-35, 40-45, 41, 53, 54, 63) being such that a combination of the at least two first scores (30-35, 40-45, 41, 53, 54, 63) provides first information on an impossibility to assess the first criterion, a presence of a defect and an absence of a defect,
• generate and output a list of global criteria (C1, C2, C3-C8,...,Cj) indicative of a health status of the organ assessed over said at least two ultrasound images (I1, ..., In), wherein each global criterion of the list of global criteria (C1, C2, C3-C8,...,Cj) is associated with at least two global scores (71-73, 81-83, 91), said at least two global scores (71-73, 81-83, 91) being such that a combination of the at least two global scores (71-73, 81-83, 91) provides a global information on a global impossibility to assess the global criterion, a global presence of a defect and a global absence of a defect, wherein the list of global criteria (C1, C2, C3-C8,...,Cj) is generated using a feed-forward network of arithmetic operators of linear or non-linear nature which maps at least two of said generated lists of first criteria (c1, c2, c3-c8,...,ci), and/or at least two lists of remarkable elements (e1, e2, e3,...,ek, 181-187) relative to the at least two ultrasound images (I1, ..., In) from which said at least two lists of first criteria have been generated, to the at least two global scores (71-73, 81-83, 91).

2. The device according to claim **1,** wherein at least one global criterion (C1, C2, C3-C8,...,Cj) is a temporal criterion indicative of a defect in a periodic evolution of the organ and the at least two global scores (71-73, 81-83, 91) are determined based on the evolution in time of the at least one remarkable element (181-187) across the at least two ultrasound images (I1, ..., In).

3. The device according to any of claims **1** or **2,** wherein each first criterion of the list of first criteria (c1, c2, c3-c8,..., ci) is further associated with a first severity score (63) representative of a magnitude of the defect.

4. The device according to any of claims **1** to **3,** wherein each global criterion of the list of global criteria (C1, C2, C3-C8,...,Cj) is further associated with a global severity score representative of a magnitude of the defect assessed on the at least two ultrasound images (I1, ..., In).

5. The device according to any of claims **1** to **4,** wherein the at least one remarkable information (10-16) is comprised in at least one remarkable score (21-27).

6. The device according to any one of claims **1** to **5,** wherein each first criterion of the list of first criteria (c1, c2, c3-c8,...,ci) is associated with three mutually exclusive first scores, including:
- an assessability score (31, 33), representative of the impossibility to assess the first criterion,
- a presence score (41, 43), representative of the presence of a defect, and
- an absence score (51, 53), representative of the absence of a defect.

7. The device according to any one of claims **1** to **5,** wherein each first criterion of the list of first criteria (c1, c2, c3-c8,...,ci) is associated with two first scores:
- a defect score (42) representative of the presence of a defect and the absence of a defect, said defect score being comprised between a lower score bound and an upper score bound, the lower score bound being representative of a high probability of an absence of a defect and the upper score bound being representative of a high probability of a presence of a defect, and
- an assessability score (32), being representative of the impossibility to assess the first criterion.

8. The device according to any one of claims **1** to **5,** wherein each global criterion of the list of global criteria (C1, C2, C3-C8,...,Cj) is associated with two global scores:
- a global defect score (72) representative of the global absence of a defect and the global presence of a defect, said global defect score being comprised between a global lower score bound and a global upper score bound, the global lower score bound being representative of a high probability of an absence of a defect across the at least two ultrasound images (I1, ..., In) and the upper score bound being representative of a high probability of a presence of a defect across the at least two ultrasound images (I1, ..., In), and
- a global assessability score (82) representative of the global impossibility to assess the global criterion.

9. The device according to any one of claims **1** to **8,** wherein the generation of the list of global criteria (C1, C2, C3-C8,...,Cj) comprises implementing a 1-dimensional convolutional neural network configured to simultaneously process the outputted lists of first criteria (c1, c2, c3-c8,...,ci) and/or the lists of remarkable elements (e1, e2, e3,...,ek, 181-187) relative to the at least two ultrasound images (I1, ..., In).

10. The device according to any one of claims **1** to **8,** wherein the generation of the list of global criteria (C1, C2, C3-C8,...,Cj) comprises implementing a recurrent convolutional neural network configured to iteratively process the outputted lists of first criteria (c1, c2, c3-c8,...,ci) and/or the lists of remarkable elements (e1, e2, e3,...,ek, 181-187) relative to the at least two ultrasound images (I1, ..., In).

11. A computer-implemented method for guiding a user in ultrasound assessment of an organ to perform a diagnosis or screening evaluation of said organ during a medical examination, said ultrasound assessment being based on at least two ultrasound image (I1, ..., In) resulting from at least a partial reflection of ultrasound waves by said organ, said method comprising:
• for each ultrasound image (I1, ..., In):
- receiving as an input, for each ultrasound image (I1, ..., In) of the at least two ultrasound images, a list of remarkable elements (e1, e2, e3,...,ek, 181-187) associated to the ultrasound image, wherein each remarkable element (e1, e2, e3,...,ek, 181-187) is associated with at least one remarkable information among:
o an observability (10) of the remarkable element (e1, e2, e3,...,ek, 181-187) or an unobservability (11) of the remarkable element (e1, e2, e3,...,ek, 181-187),
o an abnormal aspect (12) of the remarkable element (e1, e2, e3,...,ek, 181-187) or a non-abnormal aspect (13) of the remarkable element (e1, e2, e3,...,ek, 181-187)
o a normal aspect (14) of the remarkable element (e1, e2, e3,...,ek, 181-187) or a non-normal aspect (15) of the remarkable element (e1, e2, e3,...,ek, 181-187), and
o a measurement (16) performed on the remarkable element (e1, e2, e3,...,ek, 181-187),
- generating and outputting a list of first criteria (c1, c2, c3-c8,...ci) indicative of a health status of the organ, wherein each first criterion of the list of first criteria (c1, c2, c3-c8,...ci) is associated with at least two first scores (40-44, 50-54, 61, 64, 74), each first score (40-44, 50-54, 61, 64, 74) being generated using said at least one remarkable information associated to the at least one remarkable element (e1, e2, e3,...,ek, 181-187), said at least two first scores (40-44, 50-54, 61, 64, 74) being such that a combination of the at least two first scores (40-44, 50-54, 61, 64, 74) provides information on an impossibility to assess the first criterion, a presence of a defect, and an absence of a defect,
• generating and outputting a list of global criteria (C1, C2, C3-C8,...,Cj) indicative of a health status of the organ assessed over said at least two ultrasound images (I1, ..., In), wherein each global criterion of the list of global criteria (C1, C2, C3-C8,..,Cj) is associated with at least two global scores (71-73, 81-83, 91), said at least two global scores (71-73, 81-83, 91) being such that a combination of the at least two global scores (71-73, 81-83, 91) provides information on a global impossibility to assess the global criterion, a global presence of a defect and a global absence of a defect, wherein the list of global criteria (C1, C2, C3-C8,...,Cj) is generated using a feed-forward network of arithmetic operators of linear or non-linear nature which maps at least two of said generated lists of first criteria (c1, c2, c3-c8,...,ci), and/or at least two lists of remarkable elements (e1, e2, e3,...,ek, 181-187) relative to the at least two ultrasound images (I1, ..., In) from which said at least two lists of first criteria have been generated, to the at least two global scores (71-73, 81-83, 91).

12. A computer program product for guiding a user in ultrasound assessment of an organ, the computer program product comprising instructions which, when the program is executed by a computer, cause the computer to automatically carry out the method according to claim **11.**

13. A computer-readable storage medium comprising instructions which, when executed by a computer, cause the computer to carry out the method for guiding a user in ultrasound assessment of an organ according to claim **11.**

## Patentansprüche

1. Vorrichtung (1000) zum Führen eines Benutzers bei der Ultraschallbewertung eines Organs, um eine Bewertung des Organs während einer medizinischen Untersuchung durchzuführen, wobei die Ultraschallbewertung auf mindestens zwei Ultraschallbildern (I1, ..., In) basiert, die sich aus mindestens einer teilweisen Reflexion der Ultraschallwellen durch das Organ ergeben, wobei die Vorrichtung mindestens einen Prozessor umfasst, der für Folgendes konfiguriert ist:
• für jedes Ultraschallbild (I1, ..., In):
- Empfangen einer Liste von bemerkenswerten Elementen (e1, e2, e3,...,ek, 181-187), die dem Ultraschallbild (I1, ..., In) zugeordnet sind, als Eingang, wobei jedes bemerkenswerte Element (e1, e2, e3,...,ek, 181-187) mit mindestens einer bemerkenswerten Information aus dem Folgenden in Verbindung steht:
o einer Beobachtbarkeit (10) des bemerkenswerten Elements (e1, e2, e3,...,ek, 181-187) oder einer Nichtbeobachtbarkeit (11) des bemerkenswerten Elements (181-187),
o einem abnormalen Aspekt (12) des bemerkenswerten Elements (e1, e2, e3,...,ek, 181-187) oder einem nicht abnormalen Aspekt (13) des bemerkenswerten Elements (e1, e2, e3,...,ek, 181-187),
o einem normalen Aspekt (14) des bemerkenswerten Elements (e1, e2, e3,...,ek, 181-187) oder einem nicht normalen Aspekt (15) des bemerkenswerten Elements (e1, e2, e3,...,ek, 181-187), und
▪ einer Messung, die am bemerkenswerten Element (e1, e2, e3,...,ek, 181-187) durchgeführt wird,
- Erzeugen und Ausgeben einer Liste von ersten Kriterien (c1, c2, c3-c8,...ci), die auf einen Gesundheitszustand des Organs hinweisen, wobei jedes erste Kriterium der Liste der ersten Kriterien (c1, c2, c3-c8,...,ci) mit mindestens zwei ersten Scores (30-35, 40-45, 41, 53, 54, 63) in Verbindung steht, wobei jeder erste Score (30-35, 40-45, 41, 53, 54, 63) unter Verwendung der mindestens einen Information erzeugt wird, die dem mindestens einen bemerkenswerten Element (e1, e2, e3,...,ek, 181-187) zugeordnet ist, wobei die mindestens zwei ersten Scores (30-35, 40-45, 41, 53, 54, 63) derart sind, dass eine Kombination der mindestens zwei ersten Scores (30-35, 40-45, 41, 53, 54, 63) eine erste Information über eine Unmöglichkeit, das erste Kriterium zu bewerten, ein Vorhandensein eines Defekts und ein Nichtvorhandensein eines Defekts bereitstellt,
• Erzeugen und Ausgeben einer Liste von globalen Kriterien (C1, C2, C3-C8,...,Cj), die auf einen Gesundheitszustand des über die mindestens zwei Ultraschallbilder (I1, ..., In) bewerteten Organs hinweisen, wobei jedes globale Kriterium der Liste der globalen Kriterien (C1, C2, C3-C8,...,Cj) mit mindestens zwei globalen Scores (71-73, 81-83, 91) in Verbindung steht, wobei die mindestens zwei globalen Scores (71-73, 81-83, 91) derart sind, dass eine Kombination der mindestens zwei globalen Scores (71-73, 81-83, 91) eine globale Information über eine globale Unmöglichkeit, das globale Kriterium zu bewerten, ein globales Vorhandensein eines Defekts und ein globales Nichtvorhandensein eines Defekts bereitstellt, wobei die Liste der globalen Kriterien (C1, C2, C3-C8,...,Cj) unter Verwendung eines Feed-Forward-Netzwerks linearer oder nichtlinearer arithmetischer Operatoren erzeugt wird, das mindestens zwei der erzeugten Listen der ersten Kriterien (c1, c2, c3-c8,...,ci) und/oder mindestens zwei Listen der bemerkenswerten Elemente (e1, e2, e3,...,ek, 181-187) in Bezug auf die mindestens zwei Ultraschallbilder (I1, ..., In), aus denen die mindestens zwei Listen der ersten Kriterien erzeugt wurden, auf die mindestens zwei globalen Scores (71-73, 81-83, 91) abbildet.

2. Vorrichtung nach Anspruch **1,** wobei mindestens ein globales Kriterium (C1, C2, C3-C8,...,Cj) ein zeitliches Kriterium ist, das auf einen Defekt in einer periodischen Entwicklung des Organs hinweist, und die mindestens zwei globalen Scores (71-73, 81-83, 91) basierend auf der zeitlichen Entwicklung des mindestens einen bemerkenswerten Elements (181-187) über die mindestens zwei Ultraschallbilder (I1, ..., In) bestimmt werden.

3. Vorrichtung nach einem der Ansprüche **1** oder **2,** wobei jedes erste Kriterium der Liste der ersten Kriterien (c1, c2, c3-c8,..., ci) ferner mit einem ersten Schweregrad-Score (63) in Verbindung steht, der für eine Größenordnung des Defekts repräsentativ ist.

4. Vorrichtung nach einem der Ansprüche **1** bis **3,** wobei jedes globale Kriterium der Liste der globalen Kriterien (C1, C2, C3-C8,...,Cj) ferner mit einem globalen Schweregrad-Score in Verbindung steht, der für eine Größenordnung des auf den mindestens zwei Ultraschallbildern (I1, ..., In) bewerteten Defekts repräsentativ ist.

5. Vorrichtung nach einem der Ansprüche **1** bis **4,** wobei die mindestens eine bemerkenswerte Information (10-16) in mindestens einem bemerkenswerten Score (21-27) enthalten ist.

6. Vorrichtung nach einem der Ansprüche **1** bis **5,** wobei jedes erste Kriterium der Liste der ersten Kriterien (c1, c2, c3-c8,...,ci) mit drei gegenseitig ausschliesslichen ersten Scores in Verbindung steht, einschließlich:
- eines Bewertbarkeits-Scores (31, 33), der für die Unmöglichkeit, das globale Kriterium zu bewerten, repräsentativ ist,
- eines Präsenz-Scores (41, 43), der für das Vorhandensein eines Defekts repräsentativ ist, und
- eines Abwesenheits-Scores (51, 53), der für das Nichtvorhandensein eines Defekts repräsentativ ist.

7. Vorrichtung nach einem der Ansprüche **1** bis **5,** wobei jedes erste Kriterium der Liste der ersten Kriterien (c1, c2, c3-c8,...,ci) mit zwei ersten Scores in Verbindung steht:
- einem Defekt-Score (42), der für das Vorhandensein eines Defekts und das Nichtvorhandensein eines Defekts repräsentativ ist, wobei der Defekt-Score zwischen einem unteren Score-Grenzwert und einem oberen Score-Grenzwert liegt, wobei der untere Score-Grenzwert für eine hohe Wahrscheinlichkeit des Nichtvorhandenseins eines Defekts repräsentativ ist und der obere Score-Grenzwert für eine hohe Wahrscheinlichkeit des Vorhandenseins eines Defekts repräsentativ ist, und
- einem Bewertbarkeits-Score (32), der für die Unmöglichkeit, das globale Kriterium zu bewerten, repräsentativ ist.

8. Vorrichtung nach einem der Ansprüche **1** bis **5,** wobei jedes globale Kriterium der Liste der globalen Kriterien (C1, C2, C3-C8,...,Cj) mit zwei globalen Scores in Verbindung steht:
- einem globalen Defekt-Score (72), der für das globale Nichtvorhandensein eines Defekts und das globale Vorhandensein eines Defekts repräsentativ ist, wobei der globale Defekt-Score zwischen einem globalen unteren Score-Grenzwert und einem globalen oberen Score-Grenzwert liegt, wobei der globale untere Score-Grenzwert eine hohe Wahrscheinlichkeit des Nichtvorhandenseins eines Defekts über die mindestens zwei Ultraschallbilder (I1, ..., In) repräsentativ ist und der obere Score-Grenzwert für eine hohe Wahrscheinlichkeit des Vorhandenseins eines Defekts über die mindestens zwei Ultraschallbilder (I1, ..., In) repräsentativ ist, und
- eines globalen Bewertbarkeits-Scores (82), der für die globale Unmöglichkeit, das globale Kriterium zu bewerten, repräsentativ ist.

9. Vorrichtung nach einem der Ansprüche **1** bis **8,** wobei das Erzeugen der Liste der globalen Kriterien (C1, C2, C3-C8,...,Cj) das Implementieren eines 1-dimensionalen konvolutionalen neuronalen Netzwerks umfasst, das so konfiguriert ist, dass es gleichzeitig die ausgegebenen Listen der ersten Kriterien (c1, c2, c3-c8,...,ci) und/oder die Listen der bemerkenswerten Elemente (e1, e2, e3,...,ek, 181-187) in Bezug auf die mindestens zwei Ultraschallbilder (I1, ..., In) verarbeitet.

10. Vorrichtung nach einem der Ansprüche **1** bis **8,** wobei das Erzeugen der Liste der globalen Kriterien (C1, C2, C3-C8,...,Cj) das Implementieren eines wiederkehrenden konvolutionellen neuronalen Netzwerks umfasst, das so konfiguriert ist, dass es die ausgegebenen Listen der ersten Kriterien (c1, c2, c3-c8,...,ci) und/oder die Listen der bemerkenswerten Elemente (e1, e2, e3,...,ek, 181-187) in Bezug auf die mindestens zwei Ultraschallbilder (I1, ..., In) iterativ verarbeitet.

11. Computerimplementiertes Verfahren zum Führen eines Benutzers bei der Ultraschallbewertung eines Organs, um eine diagnostische oder Screening-Bewertung des Organs während einer medizinischen Untersuchung durchzuführen, wobei die Ultraschallbewertung auf mindestens zwei Ultraschallbildern (I1, ..., In) basiert, die sich aus mindestens einer teilweisen Reflexion der Ultraschallwellen durch das Organ ergeben, wobei das Verfahren Folgendes umfasst:
• für jedes Ultraschallbild (I1, ..., In):
- Empfangen einer Liste von bemerkenswerten Elementen (e1, e2, e3,...,ek, 181-187), die dem Ultraschallbild zugeordnet sind, als Eingang für jedes Ultraschallbild (I1, ..., In) der mindestens zwei Ultraschallbilder, wobei jedes bemerkenswerte Element (e1, e2, e3,...,ek, 181-187) mit mindestens einer bemerkenswerten Information aus dem Folgenden in Verbindung steht:
o einer Beobachtbarkeit (10) des bemerkenswerten Elements (e1, e2, e3,...,ek, 181-187) oder einer Nichtbeobachtung (11) des bemerkenswerten Elements (e1, e2, e3,...,ek, 181-187),
o einem abnormalen Aspekt (12) des bemerkenswerten Elements (e1, e2, e3,...,ek, 181-187) oder einem nicht abnormalen Aspekt (13) des bemerkenswerten Elements (e1, e2, e3,...,ek, 181-187)
o einem normalen Aspekt (14) des bemerkenswerten Elements (e1, e2, e3,...,ek, 181-187) oder einem nicht normalen Aspekt (15) des bemerkenswerten Elements (e1, e2, e3,...,ek, 181-187), und
o einer Messung (16), die am bemerkenswerten Element (e1, e2, e3,...,ek, 181-187) durchgeführt wird,
- Erzeugen und Ausgeben einer Liste von ersten Kriterien (c1, c2, c3-c8,...ci), die auf einen Gesundheitszustand des Organs hinweisen, wobei jedes erste Kriterium der Liste der ersten Kriterien (c1, c2, c3-c8,...ci) mit mindestens zwei ersten Scores (40-44, 50-54, 61, 64, 74) in Verbindung steht, wobei jeder erste Score (40-44, 50-54, 61, 64, 74) unter Verwendung der mindestens einen bemerkenswerten Information erzeugt wird, die dem mindestens einen bemerkenswerten Element (e1, e2, e3,...,ek, 181-187) zugeordnet ist, wobei die mindestens zwei ersten Scores (40-44, 50-54, 61, 64, 74) derart sind, dass eine Kombination der mindestens zwei ersten Punkte (40-44, 50-54, 61, 64, 74) Informationen über eine Unmöglichkeit, das erste Kriterium zu bewerten, das Vorhandensein eines Defekts und das Nichtvorhandensein eines Defekts bereitstellt,
• Erzeugen und Ausgeben einer Liste von globalen Kriterien (C1, C2, C3-C8,...,Cj), die auf einen Gesundheitszustand des über die mindestens zwei Ultraschallbilder (I1, ..., In) bewerteten Organs hinweisen, wobei jedes globale Kriterium der Liste der globalen Kriterien (C1, C2, C3-C8,...,Cj) mit mindestens zwei globalen Scores (71-73, 81-83, 91) in Verbindung steht, wobei die mindestens zwei globale Scores (71-73, 81-83, 91) derart sind, dass eine Kombination der mindestens zwei globalen Scores (71-73, 81-83, 91) Informationen über eine globale Unmöglichkeit, das globale Kriterium zu bewerten, ein globales Vorhandensein eines Defekts und ein globales Nichtvorhandensein eines Defekts bereitstellt, wobei die Liste der globalen Kriterien (C1, C2, C3-C8,...,Cj) unter Verwendung eines Feed-Forward-Netzwerks linearer oder nichtlinearer arithmetischer Operatoren erzeugt wird, das mindestens zwei der erzeugten Listen der ersten Kriterien (c1, c2, c3-c8,...,ci) und/oder mindestens zwei Listen der bemerkenswerten Elemente (e1, e2, e3,...,ek, 181-187) in Bezug auf die mindestens zwei Ultraschallbilder (I1, ..., In), aus denen die zwei Listen der ersten Kriteriene erzeugt wurden, auf die mindestens zwei Gesamtscores (71-73, 81-83, 91) abbildet.

12. Computerprogrammprodukt zum Führen eines Benutzers bei der Ultraschallbewertung eines Organs, wobei das Computerprogrammprodukt Anweisungen umfasst, die, wenn das Programm von einem Computer ausgeführt wird, den Computer dazu veranlassen, das Verfahren nach Anspruch **11** automatisch auszuführen.

13. Computerlesbares Speichermedium, das Anweisungen umfasst, die, wenn sie von einem Computer ausgeführt werden, den Computer dazu veranlassen, das Verfahren zum Führen eines Benutzers bei der Ultraschallbewertung eines Organs nach Anspruch **11** auszuführen.

## Revendications

1. Un dispositif (1000) pour guider un utilisateur dans une évaluation par ultrasons d'un organe afin d'effectuer une évaluation dudit organe au cours d'un examen médical, ladite évaluation par ultrasons étant basée sur au moins deux images échographiques (I1, ..., In) résultant d'au moins une réflexion partielle d'ondes ultrasonores par ledit organe, ledit dispositif comprenant au moins un processeur configuré pour :
• pour chaque image échographique (I1, ..., In) :
- recevoir comme entrée une liste d'éléments remarquables (e1, e2, e3,...,ek, 181-187) associés à l'image échographique (I1, ..., In), dans laquelle chaque élément remarquable (e1, e2, e3,...,ek, 181-187) est associé à au moins une information remarquable parmi :
o une observabilité (10) de l'élément remarquable (e1, e2, e3,...,ek, 181-187) ou une inobservabilité (11) de l'élément remarquable (181-187),
o un aspect anormal (12) de l'élément remarquable (e1, e2, e3,...,ek, 181-187) ou un aspect non anormal (13) de l'élément remarquable (e1, e2, e3,...,ek, 181-187),
o un aspect normal (14) de l'élément remarquable (e1, e2, e3,...,ek, 181-187) ou un aspect non normal (15) de l'élément remarquable (e1, e2, e3,...,ek, 181-187), et
o une mesure effectuée sur l'élément remarquable (e1, e2, e3,...,ek, 181-187),
- générer et fournir en sortie une liste de premiers critères (c1, c2, c3-c8,...ci) indicatifs d'un état de santé de l'organe, dans laquelle chaque premier critère de la liste de premiers critères (c1, c2, c3-c8,...,ci) est associé à au moins deux premiers scores (30-35, 40-45, 41, 53, 54, 63), chaque premier score (30-35, 40-45, 41, 53, 54, 63) étant généré à l'aide de ladite au moins une information remarquable associée audit au moins un élément remarquable (e1, e2, e3,...,ek, 181-187), lesdits au moins deux premiers scores (30-35, 40-45, 41, 53, 54, 63) étant tels qu'une combinaison des au moins deux premiers scores (30-35, 40-45, 41, 53, 54, 63) fournit une première information sur une impossibilité d'évaluer le premier critère, une présence d'un défaut et une absence d'un défaut,
• générer et fournir en sortie une liste de critères globaux (C1, C2, C3-C8,...,Cj) indicatifs d'un état de santé de l'organe évalué sur lesdites au moins deux images échographiques (I1, ..., In), où chaque critère global de la liste de critères globaux (C1, C2, C3-C8,...,Cj) est associé à au moins deux scores globaux (71-73, 81-83, 91), lesdits au moins deux scores globaux (71-73, 81-83, 91) étant tels qu'une combinaison desdits au moins deux scores globaux (71-73, 81-83, 91) fournit une information globale sur une impossibilité globale d'évaluer le critère global, une présence globale d'un défaut et une absence globale d'un défaut, où la liste de critères globaux (C1, C2, C3-C8,...,Cj) est générée à l'aide d'un réseau à propagation-avant d'opérateurs arithmétiques de nature linéaire ou non-linéaire qui fait correspondre au moins deux desdites listes générées de premiers critères (c1, c2, c3-c8,...,ci), et/ou au moins deux listes d'éléments remarquables (e1, e2, e3,...,ek, 181-187) relatives aux au moins deux images échographiques (I1, ..., In) à partir desquelles lesdites au moins deux listes de premiers critères ont été générées, aux au moins deux scores globaux (71-73, 81-83, 91).

2. Un dispositif selon la revendication **1,** dans lequel au moins un critère global (C1, C2, C3-C8,...,Cj) est un critère temporel indicatif d'un défaut dans une évolution périodique de l'organe et les au moins deux scores globaux (71-73, 81-83, 91) sont déterminés sur la base de l'évolution dans le temps du au moins un élément remarquable (181-187) à travers les au moins deux images échographiques (I1, ..., In).

3. Un dispositif selon l'une quelconque des revendications **1** ou **2,** dans lequel chaque premier critère de la liste de premiers critères (c1, c2, c3-c8,..., ci) est en outre associé à un premier score de sévérité (63) représentatif d'une magnitude du défaut.

4. Un dispositif selon l'une quelconque des revendications **1** à **3,** dans lequel chaque critère global de la liste de critères globaux (C1, C2, C3-C8,...,Cj) est en outre associé à un score global de sévérité représentatif d'une magnitude du défaut évalué sur lesdites au moins deux images échographiques (I1, ..., In).

5. Un dispositif selon l'une quelconque des revendications **1** à **4,** dans lequel ladite au moins une information remarquable (10-16) est comprise dans au moins un score remarquable (21-27).

6. Un dispositif selon l'une quelconque des revendications **1** à **5,** dans lequel chaque premier critère de la liste de premiers critères (c1, c2, c3-c8,...,ci) est associé à trois premiers scores mutuellement exclusifs, comprenant :
- un score d'évaluabilité (31, 33), représentatif de l'impossibilité d'évaluer le premier critère,
- un score de présence (41, 43), représentatif de la présence d'un défaut, et
- un score d'absence (51, 53), représentatif de l'absence d'un défaut.

7. Un dispositif selon l'une quelconque des revendications **1** à **5,** dans lequel chaque premier critère de la liste de premiers critères (c1, c2, c3-c8,...,ci) est associé à deux premiers scores :
- un score de défaut (42) représentatif de la présence d'un défaut et de l'absence d'un défaut, ledit score de défaut étant compris entre une borne inférieure de score et une borne supérieure de score, la borne inférieure de score étant représentative d'une forte probabilité d'une absence d'un défaut et la borne supérieure de score étant représentative d'une forte probabilité d'une présence d'un défaut, et
- un score d'évaluabilité (32), représentatif de l'impossibilité d'évaluer le premier critère.

8. Un dispositif selon l'une quelconque des revendications **1** à **5,** dans lequel chaque critère global de la liste de critères globaux (C1, C2, C3-C8,...,Cj) est associé à deux scores globaux :
- un score global de défaut (72) représentatif de l'absence globale d'un défaut et de la présence globale d'un défaut, ledit score global de défaut étant compris entre une borne globale inférieure de score et une borne globale supérieure de score, la borne globale inférieure de score étant représentative d'une forte probabilité d'une absence d'un défaut à travers lesdites au moins deux images échographiques (I1, ..., In) et la borne globale supérieure de score étant représentative d'une forte probabilité d'une présence d'un défaut à travers lesdites au moins deux images échographiques (I1, ..., In), et
- un score global d'évaluabilité (82) représentatif de l'impossibilité globale d'évaluer le critère global.

9. Un dispositif selon l'une quelconque des revendications **1** à **8,** dans lequel la génération de la liste de critères globaux (C1, C2, C3-C8,...,Cj) comprend l'implémentation d'un réseau neuronal convolutif unidimensionnel configuré pour traiter simultanément les listes fournies de premiers critères (c1, c2, c3-c8,...,ci) et/ou les listes d'éléments remarquables (e1, e2, e3,...,ek, 181-187) relatives aux au moins deux images échographiques (I1, ..., In).

10. Un dispositif selon l'une quelconque des revendications **1** à **8,** dans lequel la génération de la liste de critères globaux (C1, C2, C3-C8,...,Cj) comprend l'implémentation d'un réseau neuronal convolutif récurrent configuré pour traiter itérativement les listes fournies de premiers critères (c1, c2, c3-c8,...,ci) et/ou les listes d'éléments remarquables (e1, e2, e3,...,ek, 181-187) relatives aux au moins deux images échographiques (I1, ..., In).

11. Une méthode implémentée par ordinateur pour guider un utilisateur dans une évaluation par ultrasons d'un organe afin d'effectuer une évaluation dudit organe au cours d'un examen médical, ladite évaluation par ultrasons étant basée sur au moins deux images échographiques (I1, ..., In) résultant d'au moins une réflexion partielle d'ondes ultrasonores par ledit organe, ladite méthode comprenant :
• pour chaque image échographique (I1, ..., In) :
- recevoir comme entrée, pour chaque image échographique (I1, ..., In) des au moins deux images échographiques, une liste d'éléments remarquables (e1, e2, e3,...,ek, 181-187) associés à l'image échographique, chaque élément remarquable (e1, e2, e3,...,ek, 181-187) est associé à au moins une information remarquable parmi :
o une observabilité (10) de l'élément remarquable (e1, e2, e3,...,ek, 181-187) ou une inobservabilité (11) de l'élément remarquable (e1, e2, e3,...,ek, 181-187),
o un aspect anormal (12) de l'élément remarquable (e1, e2, e3,...,ek, 181-187) ou un aspect non anormal (13) de l'élément remarquable (e1, e2, e3,...,ek, 181-187),
o un aspect normal (14) de l'élément remarquable (e1, e2, e3,...,ek, 181-187) ou un aspect non normal (15) de l'élément remarquable (e1, e2, e3,...,ek, 181-187), et
o une mesure (16) effectuée sur l'élément remarquable (e1, e2, e3,...,ek, 181-187),
- générer et fournir en sortie une liste de premiers critères (c1, c2, c3-c8,...ci) indicatifs d'un état de santé de l'organe, où chaque premier critère de la liste de premiers critères (c1, c2, c3-c8,...ci) est associé à au moins deux premiers scores (40-44, 50-54, 61, 64, 74), chaque premier score (40-44, 50-54, 61, 64, 74) étant généré à l'aide de ladite au moins une information remarquable associée à au moins un élément remarquable (e1, e2, e3,...,ek, 181-187), lesdits au moins deux premiers scores (40-44, 50-54, 61, 64, 74) étant tels qu'une combinaison de au moins deux premiers scores (40-44, 50-54, 61, 64, 74) fournit une information sur une impossibilité d'évaluer le premier critère, une présence d'un défaut, et une absence d'un défaut,
• générer et fournir en sortie une liste de critères globaux (C1, C2, C3-C8,...,Cj) indicatifs d'un état de santé de l'organe évalué sur lesdites au moins deux images échographiques (I1, ..., In), où chaque critère global de la liste de critères globaux (C1, C2, C3-C8,...,Cj) est associé à au moins deux scores globaux (71-73, 81-83, 91), lesdits au moins deux scores globaux (71-73, 81-83, 91) étant tels qu'une combinaison d'au moins deux scores globaux (71-73, 81-83, 91) fournit une information sur une impossibilité globale d'évaluer le critère global, une présence globale d'un défaut et une absence globale d'un défaut, où la liste de critères globaux (C1, C2, C3-C8,...,Cj) est générée à l'aide d'un réseau à propagation-avant d'opérateurs arithmétiques de nature linéaire ou non-linéaire qui fait correspondre au moins deux desdites listes générées de premiers critères (c1, c2, c3-c8,...,ci), et/ou au moins deux listes d'éléments remarquables (e1, e2, e3,...,ek, 181-187) relatives aux au moins deux images échographiques (I1, ..., In) à partir desquelles lesdites au moins deux listes de premiers critères ont été générées, aux au moins deux scores globaux (71-73, 81-83, 91).

12. Un produit programme d'ordinateur pour guider un utilisateur dans une évaluation par ultrasons d'un organe, le produit programme d'ordinateur comprenant des instructions qui, lorsqu'elles sont exécutées par un ordinateur, amènent l'ordinateur à exécuter automatiquement la méthode selon la revendication 11.

13. Un support de stockage lisible par ordinateur comprenant des instructions qui, lorsqu'elles sont exécutées par un ordinateur, amènent l'ordinateur à exécuter la méthode pour guider un utilisateur dans une évaluation par ultrasons d'un organe selon la revendication 11.
